(19) **Europäisches Patentamt**
**European Patent Office**
**Office européen des brevets**

(11) **EP 1 386 152 B1**

(12) **EUROPÄISCHE PATENTSCHRIFT**

(45) Veröffentlichungstag und Bekanntmachung des Hinweises auf die Patenterteilung:
**21.12.2011 Patentblatt 2011/51**

(51) Int Cl.:
***G01N 33/48*** *(2006.01)*

(21) Anmeldenummer: **02732565.3**

(22) Anmeldetag: **05.04.2002**

(86) Internationale Anmeldenummer:
**PCT/EP2002/003799**

(87) Internationale Veröffentlichungsnummer:
**WO 2002/082077 (17.10.2002 Gazette 2002/42)**

(54) **HERSTELLUNG POLYKLONALER, MONOSPEZIFISCHER ANTIKÖRPER GEGEN DIE UPAR-VARIANTEN DEL4, DEL5 UND DEL4+5 SOWIE DEREN VERWENDUNG FÜR DIAGNOSTISCHE UND THERAPEUTISCHE ZWECKE**

PRODUCTION OF POLYCLONAL MONOSPECIFIC ANTIBODIES AGAINST UPAR VARIANTS DEL4, DEL5 AND DEL4+5, AND THE USE THEREOF FOR DIAGNOSTIC AND THERAPEUTICAL PURPOSES

PRODUCTION D'ANTICORPS MONOSPECIFIQUES POLYCLONAUX CONTRE LES VARIANTS UPAR DEL4, DEL5 ET DL4+5, ET LEUR UTILISATION A DES FINS DE DIAGNOSTIC ET THERAPEUTIQUES

(84) Benannte Vertragsstaaten:
**AT BE CH CY DE DK ES FI FR GB GR IE IT LI LU MC NL PT SE TR**

(30) Priorität: **06.04.2001 DE 10117381**

(43) Veröffentlichungstag der Anmeldung:
**04.02.2004 Patentblatt 2004/06**

(73) Patentinhaber: **Wilex AG**
**81675 München (DE)**

(72) Erfinder:
• **LUTHER, Thomas**
**01900 Kleinröhrsdorf (DE)**
• **MAGDOLEN, Viktor**
**85551 Kirchheim (DE)**
• **SCHMITT, Manfred**
**81669 München (DE)**
• **MÜHLENWEG, Bernd**
**80687 München (DE)**
• **KOTZSCH, Matthias**
**01326 Dresden (DE)**

(74) Vertreter: **Weiss, Wolfgang et al**
**Weickmann & Weickmann**
**Patentanwälte**
**Postfach 86 08 20**
**81635 München (DE)**

(56) Entgegenhaltungen:

• PYKE C ET AL: "AN ALTERNATIVELY SPLICED VARIANT OF MRNA FOR THE HUMAN RECEPTOR FOR UROKINASE PLASMINOGEN ACTIVATOR" FEBS LETTERS, ELSEVIER SCIENCE PUBLISHERS, AMSTERDAM, NL, Bd. 326, Nr. 1/3, 12. Juli 1993 (1993-07-12), Seiten 69-74, XP000560236 ISSN: 0014-5793
• RABBANI S A ET AL: "Isolation and characterization of multiple isoforms of the rat urokinase receptor in osteoblasts." FEBS (FEDERATION OF EUROPEAN BIOCHEMICAL SOCIETIES) LETTERS, Bd. 338, Nr. 1, 1994, Seiten 69-74, XP002231477 ISSN: 0014-5793
• CASEY JANET R ET AL: "The structure of the urokinase-type plasminogen activator receptor gene." BLOOD, Bd. 84, Nr. 4, 1994, Seiten 1151-1156, XP002231478 ISSN: 0006-4971
• HILDENBRAND RALF ET AL: "Urokinase receptor localization in breast cancer and benign lesions assessed by in situ hybridization and immunohistochemistry." HISTOCHEMISTRY AND CELL BIOLOGY, Bd. 110, Nr. 1, Juli 1998 (1998-07), Seiten 27-32, XP002231479 ISSN: 0948-6143
• KOTZSCH MATTHIAS ET AL: "New ELISA for quantitation of human urokinase receptor (CD87) in cancer." INTERNATIONAL JOURNAL OF ONCOLOGY, Bd. 17, Nr. 4, Oktober 2000 (2000-10), Seiten 827-834, XP009005890 ISSN: 1019-6439

**EP 1 386 152 B1**

• REUNING UTE ET AL: "Multifunctional potential of the plasminogen activation system in tumor invasion and metastasis (Review)." INTERNATIONAL JOURNAL OF ONCOLOGY, Bd. 13, Nr. 5, November 1998 (1998-11), Seiten 893-906, XP009006104 ISSN: 1019-6439

**Beschreibung**

**Beschreibung**

Die vorliegende Erfindung betrifft die Herstellung und Verwendung spezifischer Antikörper zum Nachweis humanen tumor-assoziierten Urokinase-Typ Plasminogenaktivator-Rezeptor (uPAR) Deletionsvarianten in biologischen Proben.

Neue Erkenntnisse zur Rolle des tumorzell-assoziierten Urokinase-Rezeptors bei Tumorinvasion und Metastasierung solider maligner Tumore sind entscheidende Voraussetzungen für die Entwicklung neuer, an der Tumorbiologie orientierter Therapiestrategien, insbesondere bei Mamma- und Ovarialkarzinomen. Sowohl das invasive Wachstum dieser Tumore als auch die Entwicklung von Tumormetastasen sind bekanntermaßen multifaktorielle Prozesse, bei denen Proteasen des Plasminogen-Aktivierungssystems, wie der Urokinase-Typ Plasminogenaktivator (uPA) und sein Inhibitor (PAI-1), eine entscheidende Rolle spielen. Der uPA-Rezeptor (uPAR; CD 87) nimmt dabei durch seine Fähigkeit, das uPA-Enzymsystem auf der Zelloberfläche zu fokussieren und zu aktivieren, eine Schlüsselposition ein. Da uPAR in Tumoren aufgrund seiner Struktur in verschiedenen molekularen Formen vorkommen kann, ist seine funktionelle und biochemische Charakterisierung im Tumorgewebe schwierig.

Der Urokinase-Rezeptor, uPAR, ein stark und heterogen glykosyliertes Protein mit einer relativen Masse von 45 - 60 kDa, ist über einen GPI-Lipidanker in der Zellmembran verankert. In der Primärstruktur des uPAR lassen sich drei homologe Domänen, die jeweils von zwei Exons des uPAR-Gens kodiert werden, unterscheiden. Die Wechselwirkung mit uPA wird vor allem durch Domäne I (DI) des uPAR vermittelt. In Domäne II (DII) und/oder Domäne III (DIII) sind jedoch weitere Determinanten für die uPA/uPAR-Interaktion lokalisiert. Mit einer weiteren Bindungsstelle (wahrscheinlich in DII/III) kann uPAR mit hoher Affinität an Vitronektin, ein Protein der extrazellulären Matrix, binden, wobei auch hier eine Wechselwirkung mit DI von Bedeutung zu sein scheint. Nach der Bindung von uPA kann die intrazelluläre Signaltransduktion offenbar über verschiedene Wege ablaufen. In der Zellmembran von aktivierten Monozyten bildet uPAR z.B. mit anderen Membran- und "second messenger"-Proteinen multimere Komplexe, die eine Signaltransduktion bewirken und auf deren funktionell Kooperation hindeuten.

Es ist bekannt, dass der uPAR nicht nur einer posttranslationalen Regulation und reversiblen Aktivierung infolge der zellulären Stimulation und Ligandenbindung unterliegt. Aufgrund seiner Struktur kann uPAR auch in verschiedenen molekularen Formen vorkommen, deren biologische Aktivität im Gewebe, vorallem bei Tumorgeschehen unterschiedlich sein könnte. Nach Abspaltung des Glykanlipidankers über zell-assoziierte Enzyme kann uPAR in seiner löslichen Form (suPAR) in Körperflüssigkeiten von Tumorpatienten nachgewiesen werden. Darüber hinaus wurden für den uPAR unterschiedliche Glykosylierungsvarianten beschrieben. So führt die Stimulation von Zellen (z.B. durch Phorbolester) zu einer deutlichen Erhöhung des Glykosylierungsgrades von uPAR. Milde Chymotrypsinandauung spaltet das uPAR Molekül zwischen DI und DII. Die resultierenden Fragmente DI und DII/III wirken in vitro bei verschiedenen Zelltypen chemotaktisch. Zum Vorkommen der uPAR Fragmente DI bzw. DII/III in vivo sowie ihrer biologischen Funktionen findet man nur wenige Aussagen in der Literatur. Beschrieben wurde bislang lediglich, dass eine DII/III uPAR-Variante (unter Abspaltung von DI) in einigen Tumorzelllinien sowie in Ovarialzystenflüssigkeit von Ovarialkarzinomen vorkommen kann. Ein tumor-selektives Vorkommen von uPAR-Deletionsvarianten ist bisher nicht bekannt. Außerdem existiert bisher kein Verfahren, welches in der Lage wäre, selektiv die uPAR-Deletionsvarianten zu detektieren.

Überraschenderweise wurde nun gefunden, dass in Tumorzellen eine selektiv erhöhte Expression von uPAR-Deletionsvarianten nachweisbar ist. Weiterhin wird ein Verfahren bereitgestellt, welches tumorassoziierte uPAR-Varianten zu charakterisieren vermag, insbesondere Deletionsvarianten des uPAR im Ovarial- und Mammakarzinom. Die durch Anwendung des Verfahrens erhaltenen Ergebnisse können in der Tumordiagnostik und insbesondere als Parameter zur Prognosebeurteilung verwendet werden. Das Verfahren umfasst vorzugsweise die Verwendung von uPAR-Antikörpern unter Bedingungen, die eine selektive Bestimmung von uPAR-Deletionsvarianten erlauben, zum Nachweis von uPAR-Varianten in biologischen Proben, z.B. in einem ELISA-Verfahren. Dabei werden vorzugsweise Antikörper eingesetzt, die selektiv an uPAR-Deletionsvarianten binden. Weiterhin werden uPAR-Deletionsvarianten als Targets für eine therapeutische Intervention und für ein Wirkstoff-Screening bereitgestellt.

Ein Gegenstand der Erfindung ist eine Nukleinsäure, kodierend für eine tumorassoziierte Deletionsvariante des humanen Urokinaserezeptors (uPAR), dadurch gekennzeichnet, dass die tumorassoziierte Deletionsvariante des humanen Urokinaserezeptors (uPAR) del4 (a) oder del4 + 5 (b) ist und dass (a) die Nukleinsäure die Nukleinsäuresequenz SEQ ID NO:1 umfasst und/oder für ein Polypeptid gemäß SEQ ID NO: 2 kodiert oder (b) die Nukleinsäure die Nukleinsäuresequenz SED ID NO: 3 umfasst und/oder für ein Polypeptid gemäß SEQ ID NO: 4 kodiert. Diese Nukleinsäuren kodieren vorzugsweise für Polypeptide, die selektiv in Tumoren und Tumorzellen exprimiert werden, insbesondere in Mamma- und Ovarialkarzinomen des Menschen. "Selektive Expression" bedeutet in diesem Zusammenhang, dass eine unterschiedliche Expressionsstärke in Tumorzellen und Normalzellen in einem geeigneten Test qualitativ oder/und quantitativ nachweisbar ist.

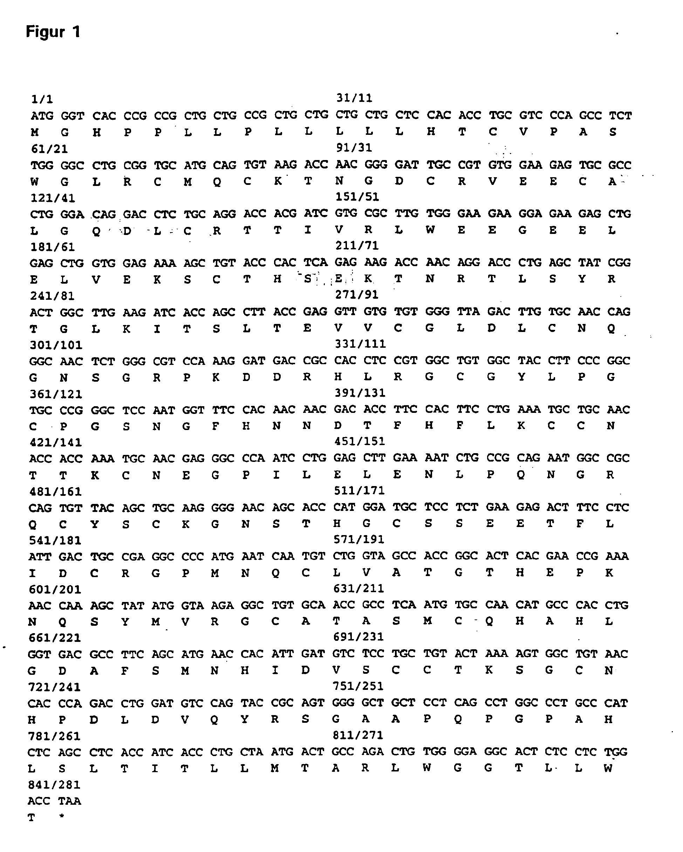

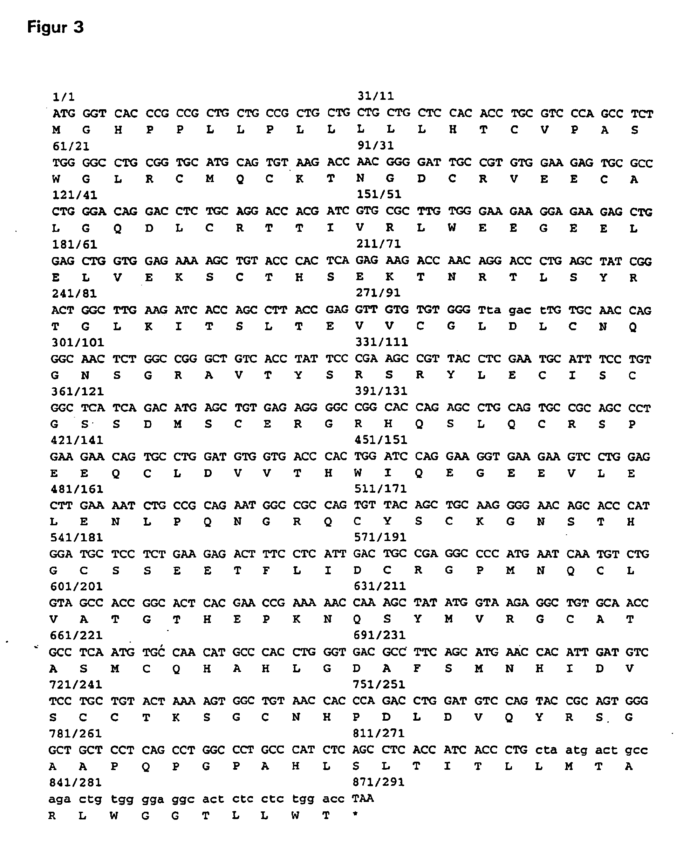

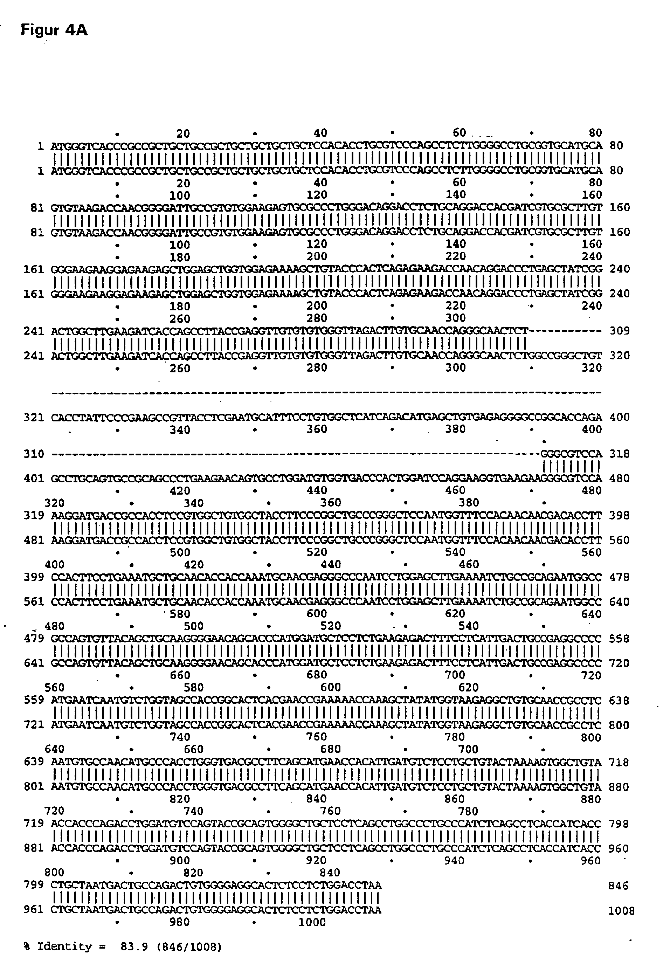

Die Figuren 1 (SEQ ID No. 1 und 2), 2 (SEQ ID No. 3 und 4) und 3 (SEQ ID No. 5 und 6) zeigen die Nukleotid- und Aminosäuresequenzen von tumorassoziierten uPAR-Deletionsvarianten del4, del4 + 5 und del 5. Die Sequenz der Deletionsvariante del5 gemäß Figur 3 (SEQ ID No. 5 und 6) ist bereits in einer öffentlich zugänglichen Datenbank (Genbank Accession Nr. U08839) beschrieben. Es findet sich jedoch dort keinerlei Hinweis auf eine tumorselektive Expression dieser Deletionsvariante. Die Figuren 4A,B, 5A,B und 6A,B zeigen einen Vergleich der Nukleotid- und Aminosäuresequenzen der Deletionsvarianten mit der entsprechenden Wildtypsequenz (SEQ ID No. 7 und 8).

Neben den Nukleotidsequenzen in den Figuren 1-2, den dazu komplementären Sequenzen und den Sequenzen die im Rahmen der Degeneration des genetischen Codes diesen Nukleotidsequenzen entsprechen, umfasst die vorliegende Erfindung auch Nukleotidsequenzen, die mit einer der zuvor genannten Sequenzen und bevorzugt mit den Fusionsübergängen hybridisieren. Vorzugsweise ist die erfindungsgemäße Nukleotidsequenz eine doppelsträngige oder einzelsträngige DNA, sie kann jedoch auch eine RNA sein. Besonders bevorzugt umfasst die erfindungsgemäße Nukleinsäure einen Abschnitt der dargestellten Nukleotidsequenzen oder eine Sequenz, die eine Identität von mehr als 80%, vorzugsweise mehr als 90% und besonders bevorzugt mehr als 95% zu einer der dargestellten Nukleotidsequenzen oder einen vorzugsweise mindestens 18 Nukleotide langen Abschnitt davon aufweist. Der Identitätsgrad I wird dabei wie folgt bestimmt:

$$I \ (\text{in } \%) \ = \ \frac{N}{L} \times 100$$

wobei N die Anzahl der übereinstimmenden Basen zwischen zu untersuchender Sequenz und Basissequenz und L die Länge der Basissequenz ist.

Erfindungsgemäße Nukleinsäuren sind vorzugsweise aus Säugern und insbesondere aus dem Menschen erhältlich, z.B. durch eine Nukleinsäure-Amplifikation, wie z.B. durch PCR. Andererseits können Nukleinsäuren auch durch rekombiante Methoden und/oder durch chemische Synthese erzeugt werden.

Erfindungsgemäße Sonden und Primer sind dadurch gekennzeichnet, dass sie selektiv die uPAR-Deletionsvarianten und nicht die native Sequenz erkennen, d.h. selektiv mit einer für eine uPAR-Deletionsvariante kodierenden Nukleinsäure hybridisieren. Sie können nach bekannten Techniken als Hybridisierungssonden und/oder als Amplifikationsprimer verwendet werden. Sie überspannen vorzugsweise einen Deletionsbereich, d.h. der Beginn der Sequenz liegt stromaufwärts des Beginns der Deletion, und das Ende der Sequenz liegt stromabwärts des Endes der Deletion.

Die Primer und Sonden sind bevorzugt mit Markern oder Markierungsgruppen versehen. Bevorzugt sind auch Primerkombinationen, die zur Identifizierung verschiedener mRNA/cDNA-Spezies geeignet sind. Besonders bevorzugt sind solche Primer, die die uPAR-Deletionsbereiche in del4 oder/und del5 oder/und del4 + 5 überspannen.

Ein weiterer Gegenstand der vorliegenden Erfindung sind tumorselektiv exprimierte uPAR-Deletionsvarianten, die von den oben definierten Nukleinsäuren kodiert sind bzw. deren Verwendung als diagnostische und therapeutische Targets. Diese Polypeptide umfassen die Aminosäuresequenzen von Figur 1 für del4 (SEQ ID No. 2) und Figur 2 für del4 + 5 (SEQ ID No. 4). Figur 3 zeigt die Aminosäuresequenz für del5 (SEQ ID No. 6).

Neben den dargestellten Polypeptidsequenzen gemäß SEQ ID NO: 2 und SEQ ID NO: 4 betrifft die Erfindung auch Varianten und Fragmente davon. Darunter sind kurze Aminosäureabschnitte von den dargestellten Aminosäuresequenzen, die eine Mindestlänge von sechs Aminosäuren, besonders bevorzugt eine Mindestlänge von acht Aminosäuren, aufweisen und Primärsequenzen aus den Deletionsbereichen, die verschieden zu der vollständigen uPAR-Primärsequenz sind, bevorzugt.

Weiterhin können erfindungsgemäße Peptide und Polypeptide auch durch chemische Synthese hergestellt werden.

Die Erfindung umfasst auch allelische Variationen oder Spleißvariationen des uPAR-Rezeptor-Proteins, sowie durch rekombinante DNA-Technologie erzeugte Proteine, die hinsichtlich ihrer biologischen und/oder immunologischen Aktivität den dargestellten Proteinen im Wesentlichen entsprechen.

Ein weiterer Gegenstand der vorliegenden Erfindung ist ein Vektor, der mindestens eine Kopie der erfindungsgemäßen Nukleinsäuren enthält. Dieser Vektor kann ein beliebiger prokaryontischer oder eukaryontischer Vektor sein, auf dem sich die erfindungsgemäße DNA-Sequenz in Verbindung mit Expressionssignalen, wie etwa Promotoren und weiteren Expressionskontrollsequenzen, befindet. Besonders bevorzugt ist der erfindungsgemäße Vektor ein eukaryontischer Vektor, z. B. ein für höhere Zellen geeigneter Vektor, z.B. ein Plasmidvektor.

Ein weiterer Gegenstand der vorliegenden Erfindung ist eine Zelle, die mit einer erfindungsgemäßen Nukleinsäure oder einem erfindungsgemäßen Vektor transformiert ist. Die Zelle kann sowohl eine eukaryontische als auch eine prokaryontische Zelle sein. Verfahren zur Transformation von Zellen mit Nukleinsäuren sind allgemeiner Stand der Technik und brauchen daher nicht näher erläutert zu werden. Beispiele für bevorzugte Zellen sind eukaryontische Zellen, insbesondere tierische Zellen und besonders bevorzugt Säugerzellen.

Ein weiterer Gegenstand der vorliegenden Erfindung sind Antikörper gegen die Deletionsvarianten des uPAR-Polypeptids. Polyklonale Antiseren erhält man durch Immunisierung von Versuchstieren mit erfindungsgemäßen Polypeptiden oder Fragmenten davon. Zur Antikörperherstellung werden vorzugsweise die erfindungsgemäßen Peptide und/oder Polypeptide an Trägerproteine wie KLH (Keyhole Limpet Hemocyanin) oder BSA (Bovine Serum Albumin) gekoppelt.

Die resultierenden Konjugate können als Immunogene zur Immunisierung von Versuchstieren verwendet werden, z.B. von Kaninchen, Hühnchen, Hamster, Ziege, Schaf und Pferd. Besonders bevorzugt werden für die Immunisierung Peptide verwendet, die den Fusionsübergängen der uPAR-Deletionsvarianten entsprechen. Die dann resultierenden polyklonalen Antikörper weisen keine Kreuzreaktivität gegen natives uPAR auf. Die Kreuzreaktivität sollte weniger als 20 % betragen, bevorzugt weniger als 10 % und besonders bevorzugt weniger als 5 % betragen.

Die erfindungsgemäßen Antiköper können auch in Form eines Konjugats mit einer Markierungsgruppe und/oder cytotoxischen Gruppe vorliegen. Als Markierungsgruppe kann ein Enzym wie alkalische Phosphatase oder Peroxidase, ein Fluoreszenzmarker, aber auch eine radioaktive Markierung verwendet werden. Als cytotoxische Gruppen können Radionuklide oder Toxine verwendet werden. Derartige Antikörper könnten dann sowohl für diagnostische Tests, insbesondere von Mamma- und Ovarialkarzinomgewebe, aber auch für die Therapie verwendet werden. Beispielsweise können mit Hilfe der ELISA-Technik Proben auf das Vorhandensein der Deletionsvarianten im Tumorgewebe untersucht werden.

Die erfindungsgemäßen selektiven Antikörper ermöglichen damit die Herstellung eines Testsystems zur prognostischen Auswertung von Tumorgewebsextrakten, um so die Rezidivhäufigkeit oder Überlebenswahrscheinlichkeit von Tumorpatienten zu bestimmen, d.h. durch die Verwendung der erfindungsgemäßen Antikörper können wertvolle Informationen zur Prognosestellung von Tumorpatienten gewonnen werden.

Insbesondere können die erfindungsgemäßen Antikörper als molekulares Nachweis-System zur Detektion von uPAR-Varianten in biologischen Proben verwendet werden.

Diagnostische Untersuchungen können auch mit Hilfe spezifischer Nukleinsäuresonden zum Nachweis auf Nukleinsäureebene, insbesondere auf Transkriptebene, durchgeführt werden. Geeignet sind die erfindungsgemäßen Sonden/Primer zur Herstellung eines Kits zur Amplifikation von uPAR-Deletionsvarianten spezifischer mRNA, z.B. in einer RT-PCR-Anwendung.

Die Erfindung betrifft weiterhin eine pharmazeutische Zusammensetzung, die als wirksame Komponente umfasst: (a) eine Nukleinsäure kodierend für eine tumorassoziierte humane uPAR-Deletionsvariante, dadurch gekennzeichnet, dass die tumorassoziierte Deletionsvariante des humanen Urokinaserezeptors (uPAR) del4 (i) oder del4 + 5 (ii) ist und dass (i) die Nukleinsäure die Nukleinsäuresequenz SEQ ID NO:1 umfasst und/oder für ein Polypeptid gemäß SEQ ID NO: 2 kodiert oder (ii) die Nukleinsäure die Nukleinsäuresequenz SED ID NO: 3 umfasst und/oder für ein Polypeptid gemäß SEQ ID NO: 4 kodiert, (b) einen Vektor, der eine Nukleinsäure gemäß (a) enthält, (c) eine Zelle, die eine Nukleinsäure gemäß (a) oder einen Vektor gemäß (b) enthält, (d) eine tumorselektiv exprimierte uPAR-Deletionsvariante, dadurch gekennzeichnet, dass sie von einer Nukleinsäure gemäß (a) kodiert wird oder (e) einen Antikörper gegen eine tumorassoziierte humane uPAR-Deletionsvariante gemäß (d), wobei der Antikörper keine Kreuzreaktivität gegen natives uPAR aufweist.

Des Weiteren betrifft ide Erfindung eine pharmazeutische Zusammensetzung als diagnostisches Mittel, die als wirksame Komponente umfasst: (a) eine Nukleinsäure kodierend für eine tumorassoziierte humane uPAR-Deletionsvariante, dadurch gekennzeichnet, dass dietumorassoziierte Deletionsvariante des humanen Urokinaserezeptors (uPAR) del4 (i) oder del4 + 5 (ii) oder del5 (iii) ist und dass (i) die Nukleinsäure die Nukleinsäuresequenz SEQ ID NO: 1 umfasst und/oder für ein Polypeptid gemäß SEQ ID NO: 2 kodiert, (ii) die Nukleinsäure die Nukleinsäuresequenz SED ID NO: 3 umfasst und/oder für ein Polypeptid gemäß SEQ ID NO: 4 kodiert oder (iii) die Nukleinsäure die Nukleinsäuresequenz SEQ ID NO: 5 umfasst und/oder für ein Polypeptid gemäß SEQ ID NO: 6 kodiert, (b) einen Vektor, der iene Nukleinsäure gemäß (a) enthält, (c) eine Zelle, die eine Nukleinsäure gemäß (a) oder einen Vektor gemäß (b) enthält, (d) eine tumorselektivexprimierteuPAR-Deletionsvariante, dadurchgekennzeichnet, dass sie von einer Nukleinsäure gemäß (a) kodiert wird oder (e) einen Antikörper gegen eine tumorassoziierte humane uPAR-Deletionsvariante gemäß (d), wobei der Antikörper keine Kreuzreaktivität gegen natives uPAR aufweist. Vorzugsweise ist diese pharmazeutische Zusammensetzung als diagnostisches Mittel, zur Prognose des Verlaufs bei Tumorerkrankungen oder zur Detektion von Tumorzellen in biologischen Proben geeignet. Besonders bevorzugt ist die Verwendung dieser pharmazeutischen Zusammensetzung, vorzugsweise eines uPAR-Deletionsvariantenspezifischen Antikörpers, als therapeutisches Mittel bei Tumorerkrankungen, wie Mamma- und Ovarialkarzinomen.

Weiterhin sind Antikörper, die gegen die Deletionsvarianten von uPAR gerichtet sind, zur Herstellung eines therapeutischen Mittels geeignet, welches beispielsweise eine selektive Funktionsblockierung bei Tumorzellen bewirken kann. Darüber hinaus können die Antikörper in Form von Konjugaten mit einer cytotoxischen Gruppe zur Wachstumshemmung oder Abtötung von Tumorzellen eingesetzt werden.

Die Anmeldung betrifft weiterhin Antisense-Nukleinsäuren, z.B. Oligodesoxynukleotide, welche den Fusionsbereich der uPAR-Varianten abdecken und gezielt zur Expressionsblockade der uPAR-Varianten eingesetzt werden können.

Ein weiterer Gegenstand der vorliegenden Erfindung ist ein Verfahren zum Nachweis von uPAR-Deletionsvarianten in Tumorzellen, dadurch gekennzeichnet, dass man (a) eine selektiv erhöhte Expression einer tumorassoziierten uPAR-Deletionsvariante del4 nachweist, die von einer Nukleinsäure kodiert wird, die die Nukleinsäuresequenz SEQ ID NO:1 umfasst und/oder für ein Polypeptid gemäß SEQ ID NO: 2 kodiert, (b) eine selektiv erhöhte Expression einer tumorassoziierten uPAR-Deletionsvariante del4 + 5 nachweist, die von einer Nukleinsäure kodiert wird, die die Nukleinsäuresequenz SED ID NO: 3 umfasst und/oder für ein Polypeptid gemäß SEQ ID NO: 4 kodiert oder (c) eine selektiv erhöhte Expression einer tumorassoziierten uPAR-Deletionsvariante del5 nachweist, die von einer Nukleinsäure kodiert wird, die die Nukleinsäuresequenz SEQ ID NO:5 umfasst und/oder für ein Polypeptid gemäß SEQ ID NO: 6 kodiert. Das erfindungsgemäße Verfahren kann vorzugsweise für die Tumordiagnostik, insbesondere für die Bestimmung von Parametern zur Prognosebeurteilung verwendet werden. Besonders bevorzugt wird das erfindungsgemäße Verfahren für die Diagnose von Ovarial- und Mammakazinom verwendet.

Ein weiterer Gegenstand der vorliegenden Erfindung ist ein Kit zum Nachweis von uPAR-Deletionsvarianten in Tumorzellen, umfassend Hybridisierungssonden oder/und Amplifikationsprimer, die selektiv mit einer fpr eine tumorassoziierte uPAR-Deletionsvariante kodierende Nukleinsäure hybridisieren und vorzugsweise einen Deletionsbereich überspannen, wobei die uPAR-Deletionsvariante kodierende Nukleinsäure (a) die Nukleinsäuresequenz SEQ ID NO:1 umfasst und/oder für ein Polypeptid gemäß SEQ ID NO: 2 kodiert, (b) die Nukleinsäuresequenz SED ID NO: 3 umfasst und/oder für ein Polypeptid gemäß SEQ ID NO: 4 kodiert oder (c) die Nukleinsäuresequenz SEQ ID NO:5 umfasst und/oder für ein Polypeptid gemäß SEQ ID NO: 6 kodiert.

Weiterhin wird die Erfindung durch folgende Figuren und Beispiele näher erläutert:

**Figur 1** zeigt die Nukleotid- und Aminosäuresequenz für die uPAR-Deletionsvariante del4 (SEQ ID No. 1 und 2).

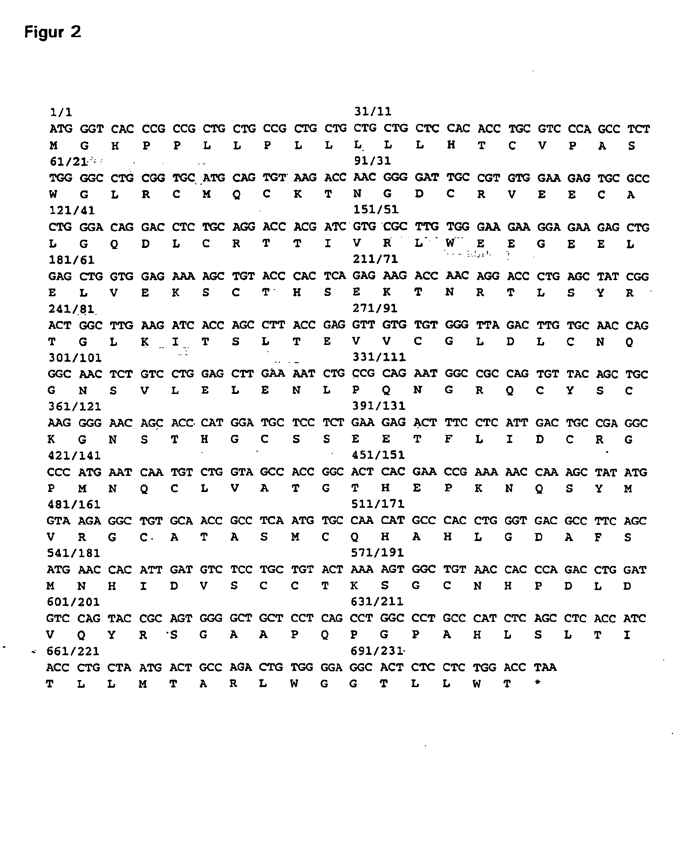

**Figur 2** zeigt die Nukleotid- und Aminosäuresequenz für die uPAR-Deletionsvariante del4 + 5 (SEQ ID No. 3 und 4).

**Figur 3** zeigt die Nukleotid-Aminosäuresequenz für die uPAR-Deletionsvariante del5 (SEQ ID No. 5 und 6).

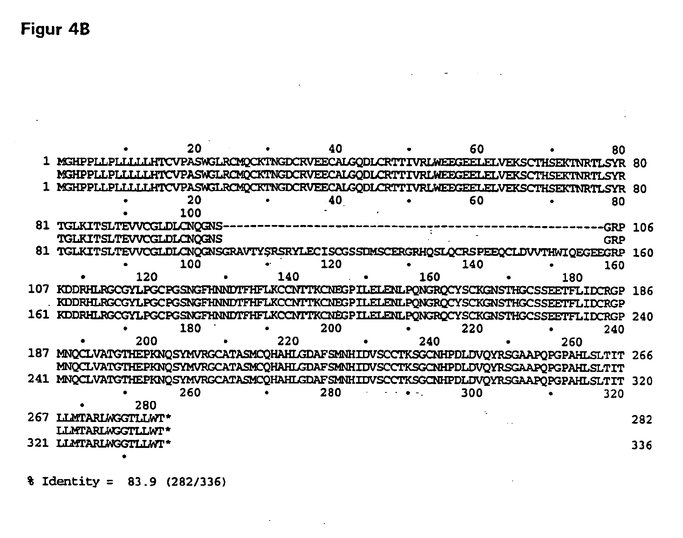

**Figur 4** zeigt einen Vergleich der Nukleotidsequenz (A) und der Aminosäuresequenz (B) mit der Wildtypsequenz (SEQ ID No. 7 und 8) für die Deletionsvariante uPAR del4.

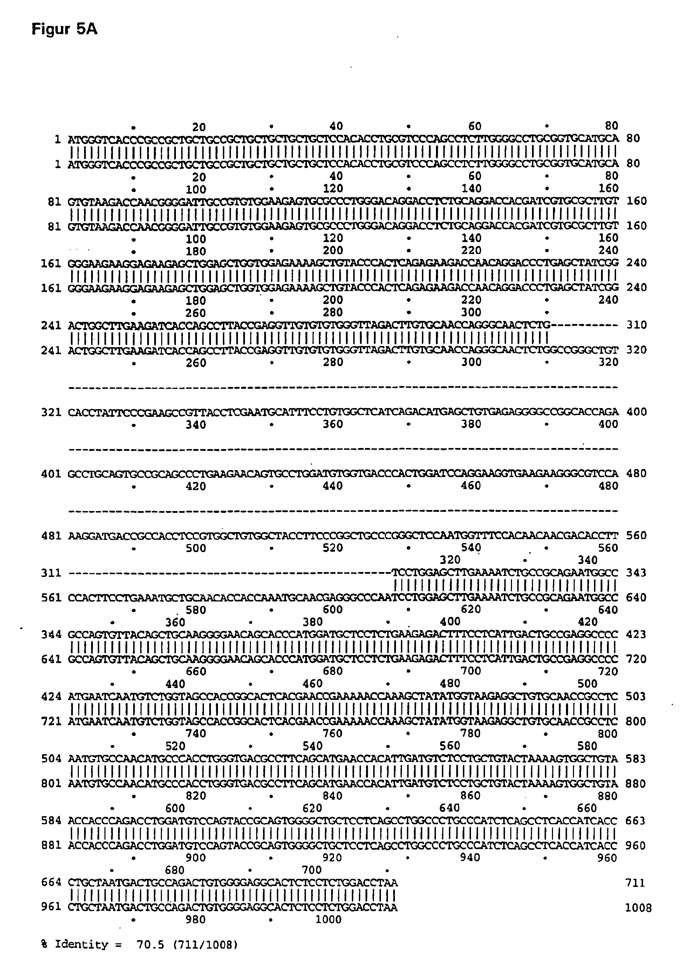

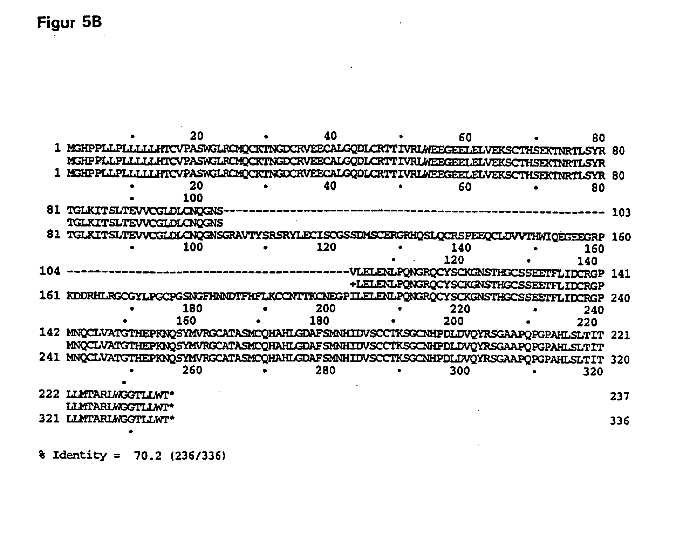

**Figur 5** zeigt einen Vergleich der Nukleotidsequenz (A) und der Aminosäuresequenz (B) mit der Wildtypsequenz (SEQ ID No. 7 und 8) für die Deletionsvariante uPAR del4 + 5.

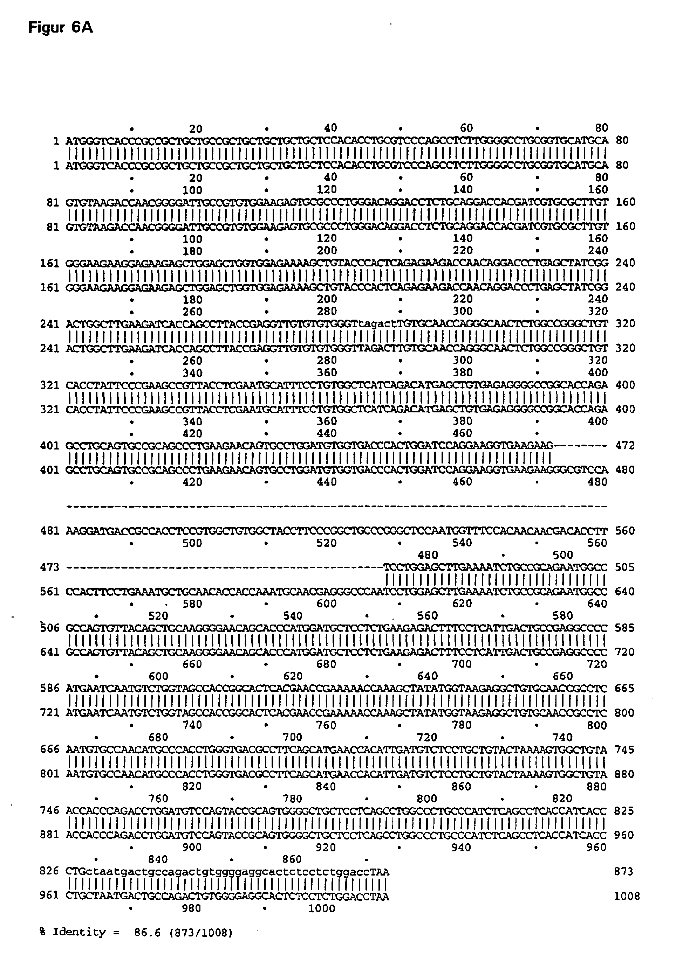

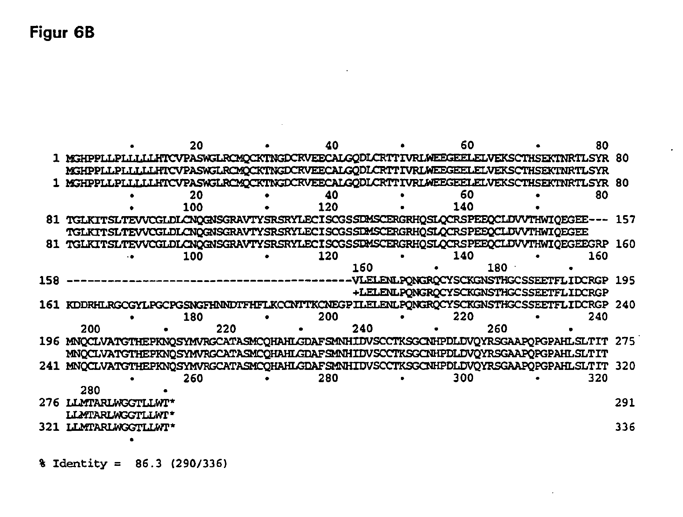

**Figur 6** zeigt einen Vergleich der Nukleotidsequenz (A) und der Aminosäuresequenz (B) mit der Wildtypsequenz (SEQ ID No. 7 und 8) für die Deletionsvariante uPAR del5.

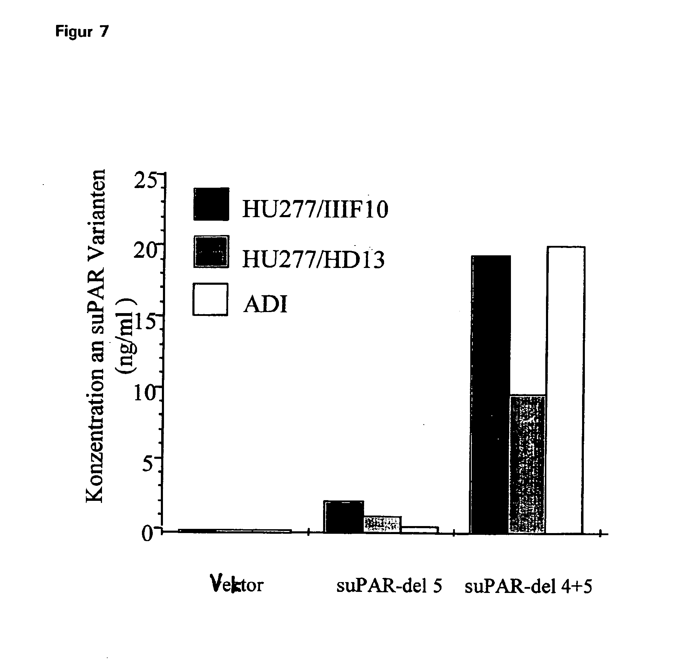

**Figur 7** zeigt die Detektion von suPAR (uPAR-Varianten) in Überständen von CHO-Zellen, die mit uPAR-Variantenkodierenden Plasmiden transfiziert wurden. HU277/IIIF10, HU277/HD13 und ADI entsprechen verschiedenen ELISA-Systemen.

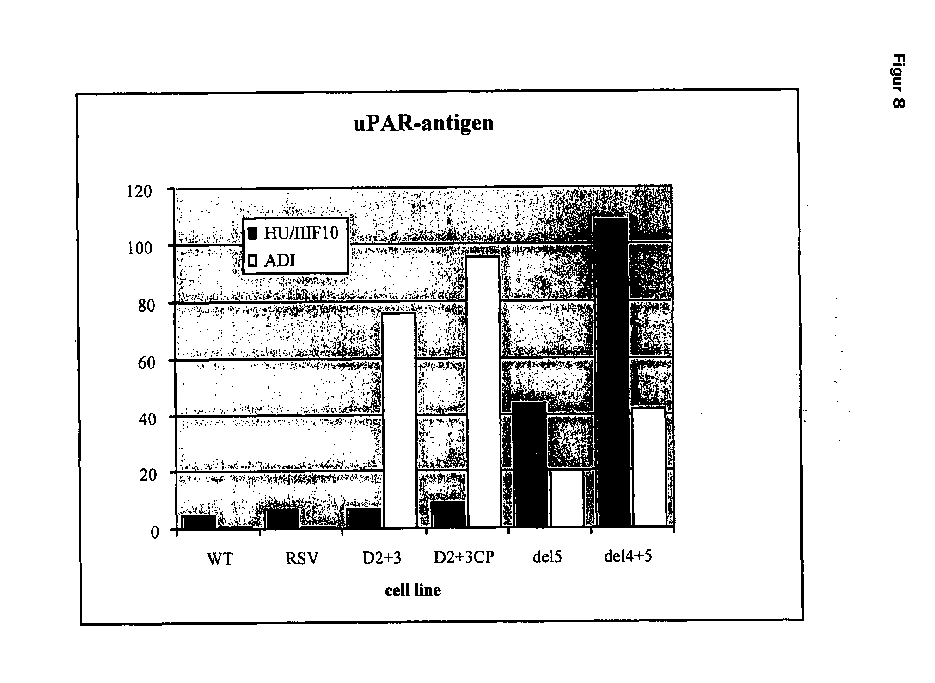

**Figur 8** zeigt die Ergebnisse der Expression von uPAR del5 und uPAR del4 + 5 als Glykophosphoinositol-verankerte Varianten mittels ELISA. Legende: Kontrollen: WT = CHO-Zellen; RSV = CHO + Vektortransfektion; D2 + 3 und D2 + 3CP = uPAR Varianten ohne Domäne 1; del5 = uPAR del5; del4 + 5 = uPAR del4 + 5

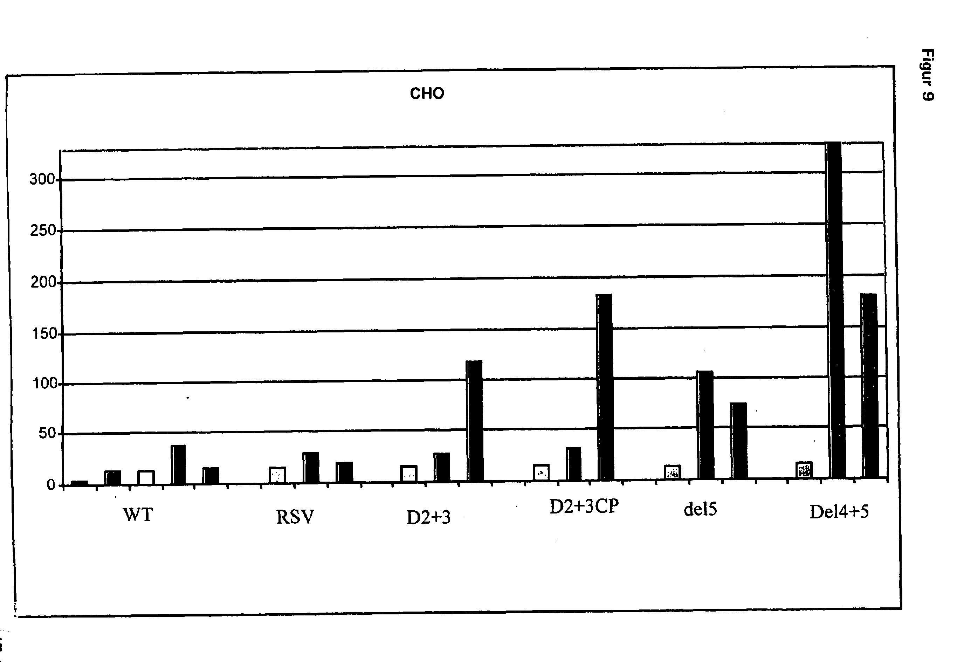

**Figur 9** zeigt die Ergebnisse von Expression der uPAR del5 und uPAR del4 + 5 als Glykophosphoinositol-verankerte Varianten mittels Durchflusszytometrie. Legende: grau = mouse IgG irrelevant; hellblau = mAb IIIF10; blau = HD13.1

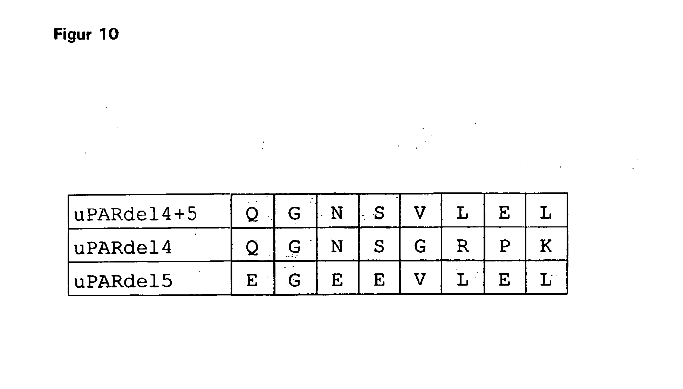

**Figur 10** zeigt Peptide, die zur Immunisierung ausgewählt wurden. Identische Aminosäuren wurden gelb hervorgehoben.

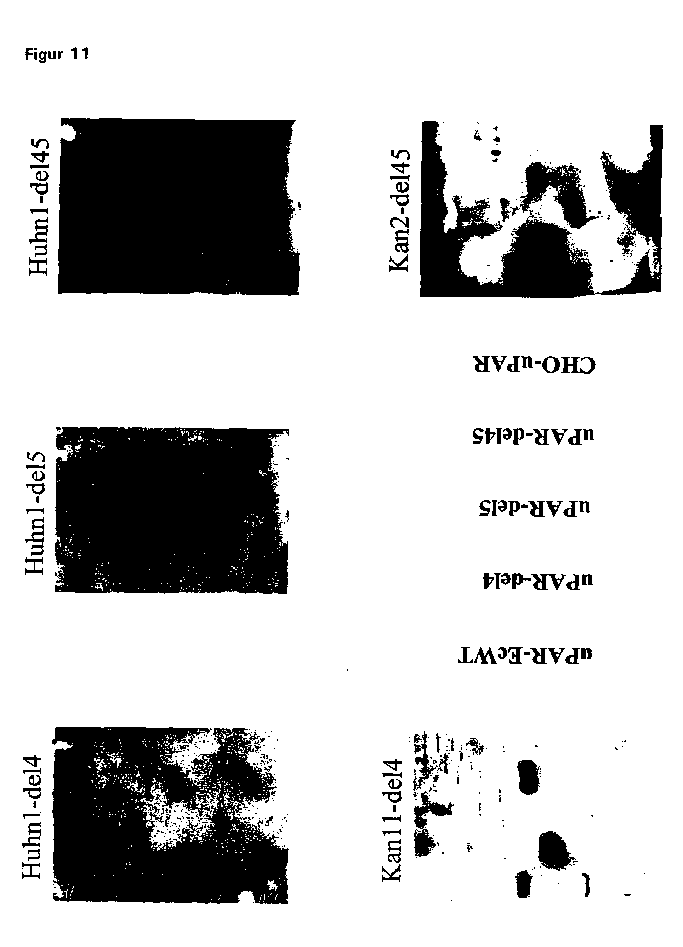

**Figur 11** zeigt die Ergebnisse der Western Blot-Analysen der gemäß Beispiel 3 isolierten peptidspezifischen Antikörper.

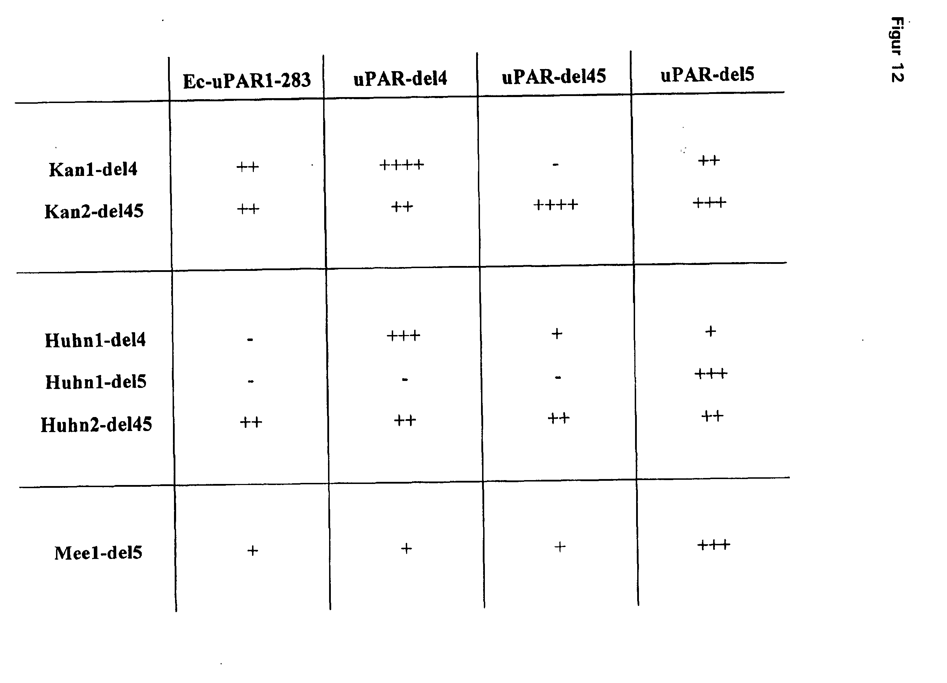

**Figur 12** zeigt eine Zusammenfassung der gemäß Beispiel 3 durchgeführten Western Blot-Analysen.

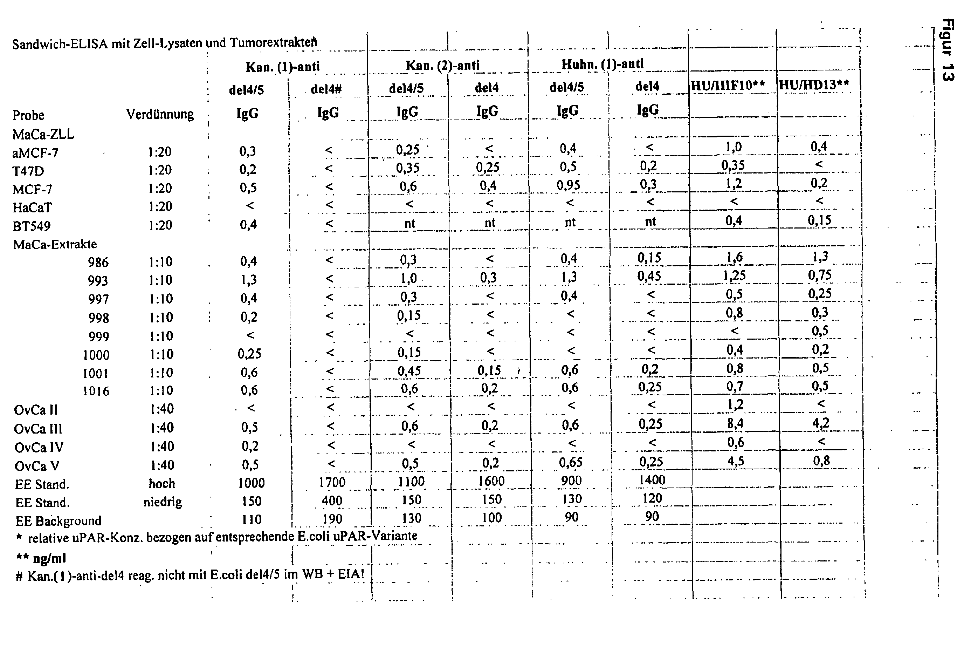

**Figur 13** zeigt die Ergebnisse von gemäß Beispiel 3 durchgeführten ELISAs.

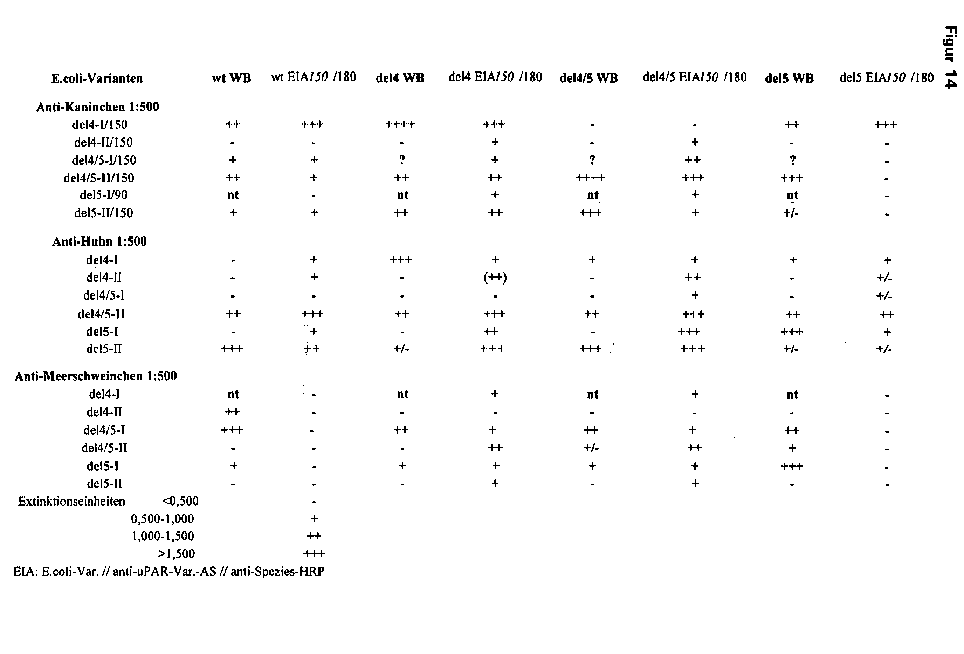

**Figur 14** zeigt einen Vergleich der Ergebnisse der gemäß Beispiel 3 durchgeführten Western Blot-Analysen und ELISAs.

**Beispiele:**

**Beispiel 1: Nachweis von mRNA-Spleiß-Varianten von uPAR**

Um zu klären, ob in Tumorzellen uPAR-mRNA-Varianten existieren, wurde mRNA aus Mammakarzinomzelllinien, aus nicht-malignen Zelllinien sowie aus Mammakarzinomgewebe isoliert und RT-PCR unter Verwendung spezifischer Primer für die uPAR-Exons 1 und 6 durchgeführt. In den nicht-malignen Zelllinien fanden sich vorwiegend Voll-Längen-uPAR-cDNA, während in Mammakarzinomzelllinien zusätzliche cDNA-Varianten mit Deletionen in Exon 4 (del4), in Exon 5 (del5) bzw. in Exon 4 + 5 (del4 + 5) nachgewiesen werden konnten. Dies konnte durch Sequenzierung der Amplifikate bestätigt werden. uPAR-Exonvarianten wurden auch im Ovarialkarzinomgewebe nachgewiesen.

**Beispiel 2: Klonierung und Expression verschiedener uPAR-Deletionsvarianten**

Verschiedene uPAR-Deletionsmutanten (Deletion-Exon-4=del4, del5, del4 + 5) wurden generiert, kloniert, sequenziert und in CHO-Zellen transfiziert (transiente Transfektion). Als Kontrollen dienten Transfektanten mit der kompletten uPAR-cDNA (D123) bzw. der cDNA für DI (D1). Die Kulturüberstände der transfizierten CHO-Zellen wurden nach 72 h abgenommen und mittels uPAR-ELISA untersucht. Alle exprimierten uPAR-Varianten (suPAR) konnten in den jeweiligen Zellkulturüberständen nachgewiesen werden, Vektorkontrolle sowie Kulturmedium waren negativ. Während die uPAR-Variante mit einer Exon 4-Deletion, sowie das komplette uPAR Molekül, von ELISA's in gleicher Weise detektiert wurden, zeigten sich im Falle des del5 und del4 + 5 Expressionsplasmids unterschiedliche Reaktionsmuster. So wurde die uPAR-del5-Variante im Gegensatz zum HU/IIIF10-ELISA in den HU/HD13 nur partiell erkannt, während die del4 + 5-Variante ausschließlich im HU/IIIF10-ELISA nachgewiesen werden konnte. Translatierte Proteine aller bekannten uPAR-mRNA Spleiß-Varianten sind mit dem HU/IIIF10-ELISA detektierbar (Figur 7).

uPAR del5 und uPAR del4 + 5 wurden zusätzlich in CHO-Zellen als Glykophosphoinositol (GPI)-verankerte Varianten exprimiert und die Expression mittels ELISA (ADI; HU/IIIF10; Figur 8) und Durchflusszytometrie (FACS-Analyse; Figur 9) nachgewiesen. D2 + 3 und D2 + 3CP stellen Kontrollvarianten des uPAR dar, denen jeweils die Domäne I des nativen Proteins fehlt. Der HU/IIIF10-ELISA kann diese Varianten nicht detektieren, während der ADI-ELISA (American Diagnostica) diese Varianten erkennt.

**Beispiel 3: Herstellung von Antikörpern**

Peptide (Figur 10) werden an das Hämocyanin einer Nacktschnecke (KLH, Keyhole Limpet Hemocyanin), mittels m-Maleimidobenzoesäure-N-hydroxysuccinimidester (MBS) gekoppelt. KLH wird im Phosphatpuffer mit MBS ca. 30 min bei Raumtemperatur leicht gerührt und überschüssiges MBS durch Gelfiltration entfernt. Danach wird Peptid in Phosphatpuffer gelöst und etwas EDTA-Lösung zugesetzt. Diese Peptidlösung wird der konzentrierten MBS-Proteinlösung zugegeben und 4 h bei Raumtemperatur leicht geschüttelt. Alternativ hierzu können die Peptide auch aus Rinderserumalbumin (BSA) nach Standardprotokollen gekoppelt werden.

Zur Immunisierung werden in der Regel Kaninchen verwendet, die Immunisierung der Kaninchen erfolgt durch subkutane Injektion von gekoppeltem Peptid und Freund'chem Adjuvanz. 4 Wochen nach der ersten Immunisierung wird die erste Booster-Injektion verabreicht. Danach wird in 2-Wochen-Abständen immunisiert und nach der zweiten Booster-Injektion, immer 10 Tage nach der Immunisierung, über die Ohrvene eine Blutprobe entnommen. Das aus dem Blut erhaltene Serum wird zur Antikörper-Titer-Bestimmung eingesetzt. Der Antikörper-Titer wird durch ein indirektes ELISA-Verfahren bestimmt.

Zusätzlich zu Kaninchen können auch Hühnchen und Meerschweinchen zur Gewinnung polyklonaler Antiseren nach Standardprotokollen verwendet werden. Hier wurden jeweils 6 Tiere jeder Spezies (Kaninchen, Hühnchen und Meerschweinchen) entsprechend durch mehrere Injektionen immunisiert, um nach 180 Tagen die Immunisierung zur Gewinnung von Serum/Eiern zu beenden.

Die erhaltenen Seren und Eier wurden zur Isolierung der peptidspezifischen Antikörper verwendet und diese in Western Blot-Analysen und ELISA-Verfahren getestet.

Für die Charakterisierung der uPAR-Varianten wurden die entsprechenden uPAR del4, uPAR del5, uPAR del4 + 5 sowie Wildtyp (WT)-uPAR als nichtglykosilierte Proteine in E.coli produziert (Expressionsvektor pQE30). Weiterhin wurde glykosilierter WT-uPAR aus Hamster-Zellen (CHO-uPAR) verwendet.

Fünf von 18 Antikörpern zeigten gute Reaktivität. Dies waren Kan1-del4; Kan2-del4 + 5; Huhn1-del5; Huhn1-del4 und Huhn1-del4 + 5. Diese Antiseren zeigten keine Reaktion mit CHO-uPAR (Figuren 11, 12, 14).

ELISA-Untersuchungen belegen die Verwendbarkeit der erhaltenen Antikörper auch zur Analyse von uPAR-Varianten in Tumorextrakten (Figuren 13,14).

**Beispiel 4: Immunhistochemie**

Immunhistochemische Untersuchungen wurden nach Standardprotokollen an Mammakarzinom-Gewebeschnitten durchgeführt. Getestet wurden die Antiseren Kan 1-del4 + 5; Kan2-del4 + 5; Kan1-del4 und Huhn2-del4 + 5.

Huhn2-del4 + 5 reagiert nach Mikrowellenbehandlung auch mit glykosyliertem WT-uPAR, aber auch zusätzliche Färbungen sind in der Tumorzelle aufgrund der Färbung von uPAR-del4 + 5 erhalten worden. Kan 1-del4 + 5; Kan2-del4 + 5 ergeben ein ähnliches Färbungsmuster.

**Patentansprüche**

1.  Nukleinsäure kodierend für eine tumorassoziierte Deletionsvariante des humanen Urokinaserezeptors (uPAR)
    **dadurch gekennzeichnet,**
    **dass** die tumorassoziierte Deletionsvariante des humanen Urokinaserezeptors (uPAR) del4 (a) oder del4 + 5 (b) ist und dass

    (a) die Nukleinsäure die Nukleotidsequenz SEQ ID NO: 1 umfasst und/oder für ein Polypeptid gemäß SEQ ID NO: 2 kodiert oder
    (b) die Nukleinsäure die Nukleotidsequenz SEQ ID NO: 3 umfasst und/oder für ein Polypeptid gemäß SEQ ID NO: 4 kodiert.

2.  uPAR-spezifisches Oligonukleotid,
    **dadurch gekennzeichnet,**
    **dass** es selektiv mit einer für eine uPAR-Deletionsvariante kodierenden Nukleinsäure nach Anspruch 1 hybridisiert.

3.  Rekombinanter Vektor,

**dadurch gekennzeichnet,**
**dass** er in operativer Verknüpfung mit einer Expressions-Kontrollsequenz mindestens eine Kopie einer Nukleinsäure nach Anspruch 1 aufweist.

4. Zelle,
   **dadurch gekennzeichnet,**
   **dass** sie mit einer Nukleinsäure nach Anspruch 1 oder einem Vektor nach Anspruch 3 transformiert ist.

5. Tumorselektiv exprimierte uPAR-Deletionsvariante,
   **dadurch gekennzeichnet,**
   **dass** sie von einer Nukleinsäure nach Anspruch 1 kodiert ist.

6. Fragment einer tumorassoziierten humanen uPAR-Deletionsvariante nach Anspruch 5,
   **dadurch gekennzeichnet,**
   **dass** es eine Mindestlänge von 6 Aminosäuren aufweist und eine für einen uPAR-Deletionsbereich charakteristische Aminosäuresequenz verschieden von der nativen uPAR-Aminosäuresequenz aufweist.

7. Verwendung einer tumorassoziierten humanen uPAR-Deletionsvariante nach Anspruch 5 oder eines Fragments davon nach Anspruch 6, als Immunogen zur Herstellung von Antikörpern.

8. Antikörper gegen eine tumorassoziierte humane uPAR-Deletionsvariante nach Anspruch 5,
   **dadurch gekennzeichnet,**
   **dass** er keine Kreuzreaktivität gegen den nativen uPAR aufweist.

9. Pharmazeutische Zusammensetzung, die als wirksame Komponente umfasst:

   (a) eine Nukleinsäure kodierend für eine tumorassoziierte humane uPAR-Deletionsvariante,
   **dadurch gekennzeichnet,**
   **dass** die tumorassoziierte Deletionsvariante des humanen Urokinaserezeptors (uPAR) del4 (i) oder del4 + 5 (ii) ist und dass

   (i) die Nukleinsäure die Nukleotidsequenz SEQ ID NO: 1 umfasst und/oder für ein Polypeptid gemäß SEQ ID NO: 2 kodiert oder
   (ii) die Nukleinsäure die Nukleotidsequenz SEQ ID NO: 3 umfasst und/oder für ein Polypeptid gemäß SEQ ID NO: 4 kodiert,

   (b) einen Vektor, der eine Nukleinsäure gemäß (a) enthält,
   (c) Eine Zelle, die eine Nukleinsäure gemäß (a) oder einen Vektor gemäß (b) enthält,
   (d) eine tumorselektiv exprimierte uPAR-Deletionsvariante,
   **dadurch gekennzeichnet, dass** sie von einer Nukleinsäure gemäß (a) kodiert wird oder
   (e) einen Antikörper gegen eine tumorassoziierte humane uPAR-Deletionsvariante gemäß (d), wobei der Antikörper keine Kreuzreaktivität gegen natives uPAR aufweist.

10. Pharmazeutische Zusammensetzung als diagnostisches Mittel, die als wirksame Komponente umfasst:

    (a) eine Nukleinsäure kodierend für eine tumorassoziierte humane uPAR-Deletionsvariante,
    **dadurch gekennzeichnet,**
    **dass** die tumorassoziierte Deletionsvariante des humanen Urokinaserezeptors (uPAR) del4 (i) oder del4 + 5 (ii) oder del 5 (iii) ist und dass

    (i) die Nukleinsäure die Nukleotidsequenz SEQ ID NO: 1 umfasst und/oder für ein Polypeptid gemäß SEQ ID NO: 2 kodiert,
    (ii) die Nukleinsäure die Nukleotidsequenz SEQ ID NO: 3 umfasst und/oder für ein Polypeptid gemäß SEQ ID NO: 4 kodiert oder
    (iii) die Nukleinsäure die Nukleotidsequenz SEQ ID NO: 5 umfasst und/oder für ein Polypeptid gemäß SEQ ID NO: 6 kodiert,

    (b) einen Vektor, der eine Nukleinsäure gemäß (a) enthält,

(c) eine Zelle, die eine Nukleinsäure gemäß (a) oder einen Vektor gemäß (b) enthält,

(d) eine tumorselektiv exprimierte uPAR-Deletionsvariante,

**dadurch gekennzeichnet, dass** sie von einer Nukleinsäure gemäß (a) kodiert wird oder

(e) einen Antikörper gegen eine tumorassoziierte humane uPAR-Deletionsvariante gemäß (d), wobei der Antikörper keine Kreuzreaktivität gegen natives uPAR aufweist.

**11.** Zusammensetzung nach Anspruch 10 zur Prognose des Verlaufs bei Tumorerkrankungen oder zur Detektion von Tumorzellen in biologischen Proben.

**12.** Antikörper nach Anspruch 8 als molekulares Nachweis-System zur Detektion von uPAR-Varianten in biologischen Proben.

**13.** Verfahren zum Nachweis von uPAR-Deletionsvarianten in Tumorzellen,
**dadurch gekennzeichnet,**
**dass** man

(a) eine selektiv erhöhte Expression einer tumorassoziierten uPAR-Deletionsvariante del4 nachweist, die von einer Nukleinsäure kodiert wird, die die Nukleotidsequenz SEQ ID NO: 1 umfasst und/oder für ein Polypeptid gemäß SEQ ID NO: 2 kodiert,

(b) eine selektiv erhöhte Expression einer tumorassoziierten uPAR-Deletionsvariante del4 + 5 nachweist, die von einer Nukleinsäure kodiert wird, die die Nukleotidsequenz SEQ ID NO: 3 umfasst und/oder für ein Polypeptid gemäß SEQ ID NO: 4 kodiert oder

(c) eine selektiv erhöhte Expression einer tumorassoziierten uPAR-Deletionsvariante del5 nachweist, die von einer Nukleinsäure kodiert wird, die die Nukleotidsequenz SEQ ID NO: 5 umfasst und/oder für ein Polypeptid gemäß SEQ ID NO: 6 kodiert.

**14.** Verfahren nach Anspruch 13 für die Tumordiagnostik, insbesondere für die Bestimmung von Parametern zur Prognosebeurteilung.

**15.** Verfahren nach Anspruch 13 oder 14 für die Diagnose von Ovarial- und Mammakarzinom.

**16.** Kit zum Nachweis von uPAR-Deletionsvarianten in Tumorzellen, umfassend Hybridisierungssonden oder/und Amplifikationsprimer, die selektiv mit einer für eine tumorassoziierte uPAR-Deletionsvariante kodierende Nukleinsäure hybridisieren und vorzugsweise einen Deletionsbereich überspannen, wobei die uPAR-Deletionsvariante kodierende Nukleinsäure

(a) die Nukleotidsequenz SEQ ID NO: 1 umfasst und/oder für ein Polypeptid gemäß SEQ ID NO: 2 kodiert,

(b) die Nukleotidsequenz SEQ ID NO: 3 umfasst und/oder für ein Polypeptid gemäß SEQ ID NO: 4 kodiert oder

(c) die Nukleotidsequenz SEQ ID NO: 5 umfasst und/oder für ein Polypeptid gemäß SEQ ID NO: 6 kodiert

**Claims**

**1.** A nucleic acid which encodes a tumour-associated deletion variant of the human urokinase receptor (uPAR)
**characterised in that**
the tumour-associated deletion variant of the human urokinase receptor (uPAR) is del4 (a) or del4+5 (b) and **in that**

(a) the nucleic acid comprises the nucleotide sequence SEQ ID no. 1 and/or encodes a polypeptide according to SEQ ID no. 2 or

(b) the nucleic acid comprises the nucleotide sequence SEQ ID no. 3 and/or encodes a polypeptide according to SEQ ID no. 4.

**2.** A uPAR-specific oligonucleotide,
**characterised in that**
it selectively hybridises with a nucleic acid which encodes a uPAR deletion variant according to claim 1.

**3.** A recombinant vector,
**characterised in that**

it comprises at least one copy of a nucleic acid according to claim 1 in operative linkage with an expression control sequence.

4. A cell,
   **characterised in that**
   it is transformed with a nucleic acid according to claim 1 or a vector according to claim 3.

5. A tumour-selectively expressed uPAR deletion variant,
   **characterised in that**
   it is encoded by a nucleic acid according to claim 1.

6. A fragment of a tumour-associated human uPAR deletion variant according to claim 5,
   **characterised in that**
   it has a minimum length of 6 amino acids and comprises an amino acid sequence characteristic of a uPAR deletion region which differs from the native uPAR amino acid sequence.

7. Use of a tumour-associated human uPAR deletion variant according to claim 5 or of a fragment thereof according to claim 6 as an immunogen for producing antibodies.

8. An antibody against a tumour-associated human uPAR deletion variant according to claim 5,
   **characterised in that**
   it does not exhibit cross-reactivity against the native uPAR.

9. A pharmaceutical composition comprising as active component:

   (a) a nucleic acid which encodes a tumour-associated human uPAR deletion variant,
   **characterised in that**
   the tumour-associated deletion variant of the human urokinase receptor (uPAR) is del4 (i) or del4+5 (ii) and **in that**

   (i) the nucleic acid comprises the nucleotide sequence SEQ ID no. 1 and/or encodes a polypeptide according to SEQ ID no. 2 or
   (ii) the nucleic acid comprises the nucleotide sequence SEQ ID no. 3 and/or encodes a polypeptide according to SEQ ID no. 4,

   (b) a vector which contains a nucleic acid according to (a),
   (c) a cell which contains a nucleic acid according to (a) or a vector according to (b),
   (d) a tumour-selectively expressed uPAR deletion variant,
   **characterised in that** it is encoded by a nucleic acid according to (a) or
   (e) an antibody against a tumour-associated human uPAR deletion variant according to (d), wherein the antibody does not exhibit cross-reactivity against native uPAR.

10. A pharmaceutical composition as a diagnostic agent which comprises as active component:

    (a) a nucleic acid which encodes a tumour-associated human uPAR deletion variant,
    **characterised in that**
    the tumour-associated deletion variant of the human urokinase receptor (uPAR) is del4 (i) or del4+5 (ii) or del5 (iii) and **in that**

    (i) the nucleic acid comprises the nucleotide sequence SEQ ID no. 1 and/or encodes a polypeptide according to SEQ ID no. 2,
    (ii) the nucleic acid comprises the nucleotide sequence SEQ ID no. 3 and/or encodes a polypeptide according to SEQ ID no. 4 or
    (iii) the nucleic acid comprises the nucleotide sequence SEQ ID no. 5 and/or encodes a polypeptide according to SEQ ID no. 6,

    (b) a vector which contains a nucleic acid according to (a),
    (c) a cell which contains a nucleic acid according to (a) or a vector according to (b),
    (d) a tumour-selectively expressed uPAR deletion variant,

**characterised in that** it is encoded by a nucleic acid according to (a) or
(e) an antibody against a tumour-associated human uPAR deletion variant according to (d), wherein the antibody does not exhibit cross-reactivity against native uPAR.

**11.** A composition according to claim 10 for predicting the course of tumour diseases or for detecting tumour cells in biological samples.

**12.** An antibody according to claim 8 as a molecular detection system for detecting uPAR variants in biological samples.

**13.** A method for detecting uPAR deletion variants in tumour cells,
**characterised in that**

(a) selectively increased expression of a tumour-associated uPAR deletion variant del4 is detected, which deletion variant is encoded by a nucleic acid which comprises the nucleotide sequence SEQ ID no. 1 and/or encodes a polypeptide according to SEQ ID no. 2,
(b) selectively increased expression of a tumour-associated uPAR deletion variant del4+5 is detected, which deletion variant is encoded by a nucleic acid which comprises the nucleotide sequence SEQ ID no. 3 and/or encodes a polypeptide according to SEQ ID no. 4 or
(c) selectively increased expression of a tumour-associated uPAR deletion variant del5 is detected, which deletion variant is encoded by a nucleic acid which comprises the nucleotide sequence SEQ ID no. 5 and/or encodes a polypeptide according to SEQ ID no. 6.

**14.** A method according to claim 13 for tumour diagnosis, in particular for determining parameters for prognosis assessment.

**15.** A method according to claim 13 or claim 14 for diagnosing ovarian and mammary carcinoma.

**16.** A kit for detecting uPAR deletion variants in tumour cells, comprising hybridisation probes and/or amplification primers which selectively hybridise with a nucleic acid which encodes a tumour-associated uPAR deletion variant and preferably span a deletion region, wherein the nucleic acid which encodes the uPAR deletion variant

(a) comprises the nucleotide sequence SEQ ID no. 1 and/or encodes a polypeptide according to SEQ ID no. 2,
(b) comprises the nucleotide sequence SEQ ID no. 3 and/or encodes a polypeptide according to SEQ ID no. 4 or
(c) comprises the nucleotide sequence SEQ ID no. 5 and/or encodes a polypeptide according to SEQ ID no. 6.

**Revendications**

**1.** Acide nucléique codant pour une variante de délétion associée à une tumeur du récepteur d'urokinase humain (uPAR), **caractérisé en ce que** la variante de délétion associée à une tumeur du récepteur d'urokinase humain (uPAR) est del4(a) ou del4+5 (b) et **en ce que**

(a) l'acide nucléique comprend la séquence de nucléotides SEQ ID NO: 1 et/ou code pour un polypeptide correspondant à SEQ ID NO: 2, ou
(b) l'acide nucléique comprend la séquence de nucléotides SEQ ID NO: 3 et/ou code pour un polypeptide correspondant à SEQ ID NO: 4.

**2.** Oligonucléotide spécifique de l'uPAR, **caractérisé en ce qu'**il s'hybride de façon sélective avec un acide nucléique codant pour une variante de délétion de l'uPAR selon la revendication 1.

**3.** Vecteur recombiné, **caractérisé en ce qu'**il présente au moins une copie d'un acide nucléique selon la revendication 1 en liaison opérationnelle avec une séquence de contrôle de l'expression.

**4.** Cellule, **caractérisée en ce qu'**elle est transformée avec un acide nucléique selon la revendication 1 ou un vecteur selon la revendication 3.

**5.** Variante de délétion de l'uPAR exprimée de façon sélective d'une tumeur, **caractérisée en ce qu'**elle est codée par un acide nucléique selon la revendication 1.

**6.** Fragment d'une variante de délétion de l'uPAR humain associée à une tumeur selon la revendication 5, **caractérisé en ce qu'**il présente une longueur d'au moins 6 aminoacides et une séquence d'aminoacides caractéristique pour un domaine de délétion de l'uPAR différente de la séquence d'aminoacides de l'uPAR natif.

**7.** Utilisation d'une variante de délétion de l'uPAR humain associée à une tumeur selon la revendication 5 ou d'un de ses fragments selon la revendication 6 comme immunogène pour la préparation d'anticorps.

**8.** Anticorps dirigé contre une variante de délétion de l'uPAR humain associée à une tumeur selon la revendication 5, **caractérisé en ce qu'**il ne présente pas de réaction croisée contre l'uPAR natif.

**9.** Composition pharmaceutique comprenant comme constituant actif:

(a) un acide nucléique codant pour une variante de délétion de l'uPAR humain associée à une tumeur, **caractérisé en ce que**
la variante de délétion associée à une tumeur du récepteur d'urokinase humain (uPAR) est del4 (i) ou del4+5 (ii) et **en ce que**

(i) l'acide nucléique comprend la séquence de nucléotides SEQ ID NO: 1 et/ou code pour un polypeptide correspondant à SEQ ID NO: 2, ou
(ii) l'acide nucléique comprend la séquence de nucléotides SEQ ID NO: 3 et/ou code pour un polypeptide correspondant à SEQ ID NO: 4,

(b) un vecteur contenant un acide nucléique selon (a),
(c) une cellule contenant un acide nucléique selon (a) ou un vecteur selon (b),
(d) une variante de délétion de l'uPAR exprimée de façon sélective d'une tumeur, **caractérisée en ce qu'**elle est codée par un acide nucléique selon (a), ou
(e) un anticorps dirigé contre une variante de délétion de l'uPAR humain associée à une tumeur selon (d), l'anticorps ne présentant pas de réaction croisée contre l'uPAR natif.

**10.** Composition pharmaceutique en tant qu'agent de diagnostic, comprenant comme constituant actif:

(a) un acide nucléique codant pour une variante de délétion de l'uPAR humain associée à une tumeur, **caractérisé en ce que**
la variante de délétion associée à une tumeur du récepteur d'urokinase humain (uPAR) est del4 (i) ou del4+5 (ii) ou del5 (iii) et **en ce que**

(i) l'acide nucléique comprend la séquence de nucléotides SEQ ID NO: 1 et/ou code pour un polypeptide correspondant à SEQ ID NO: 2, ou
(ii) l'acide nucléique comprend la séquence de nucléotides SEQ ID NO: 3 et/ou code pour un polypeptide correspondant à SEQ ID NO: 4,
(iii) l'acide nucléique comprend la séquence de nucléotides SEQ ID NO: 5 et/ou code pour un polypeptide correspondant à SEQ ID NO: 6,

(b) un vecteur contenant un acide nucléique selon (a),
(c) une cellule contenant un acide nucléique selon (a) ou un vecteur selon (b),
(d) une variante de délétion de l'uPAR exprimée de façon sélective d'une tumeur, **caractérisée en ce qu'**elle est codée par un acide nucléique selon (a), ou
(e) un anticorps dirigé contre une variante de délétion de l'uPAR humain associée à une tumeur selon (d), l'anticorps ne présentant pas de réaction croisée contre l'uPAR natif.

**11.** Composition selon la revendication 10 destinée au pronostic de l'évolution dans des maladies tumorales ou à la détection de cellules tumorales dans des échantillons biologiques.

**12.** Anticorps selon la revendication 8 en tant que système de détection moléculaire pour la détection de variante de l'uPAR dans des échantillons biologiques.

**13.** Procédé de détection de variantes de délétion de l'uPAR dans des cellules tumorales, **caractérisé en ce que**

(a) on détecte une expression sélectivement accrue d'une variante de délétion de l'uPAR del4 associée à une tumeur, qui est codée par un acide nucléique qui comprend la séquence de nucléotides SEQ ID NO: 1 et/ou code pour un polypeptide correspondant à SEQ ID NO: 2,

(b) on détecte une expression sélectivement accrue d'une variante de délétion de l'uPAR del4+5 associée à une tumeur, qui est codée par un acide nucléique qui comprend la séquence de nucléotides SEQ ID NO: 3 et/ou code pour un polypeptide correspondant à SEQ ID NO: 4, ou

(c) on détecte une expression sélectivement accrue d'une variante de délétion de l'uPAR del5 associée à une tumeur, qui est codée par un acide nucléique qui comprend la séquence de nucléotides SEQ ID NO: 5 et/ou code pour un polypeptide correspondant à SEQ ID NO: 6.

14. Procédé selon la revendication 13, destiné au diagnostic de tumeurs, en particulier à la détermination de paramètres pour l'évaluation du pronostic.

15. Procédé selon la revendication 13 ou 14, destiné au diagnostic du cancer de l'ovaire et du sein.

16. Kit de détection de variantes de délétion de l'uPAR dans des cellules tumorales, comprenant des sondes d'hybridation et/ou des amorces d'amplification qui s'hybrident de façon sélective avec un acide nucléique codant pour une variante de délétion de l'uPAR associée à une tumeur et recouvrent de préférence un domaine de délétion, où l'acide nucléique codant pour une variante de délétion de l'uPAR

(a) comprend la séquence de nucléotides SEQ ID NO: 1 et/ou code pour un polypeptide correspondant à SEQ ID NO: 2,

(b) comprend la séquence de nucléotides SEQ ID NO: 3 et/ou code pour un polypeptide correspondant à SEQ ID NO: 4, ou

(c) comprend la séquence de nucléotides SEQ ID NO: 5 et/ou code pour un polypeptide correspondant à SEQ ID NO: 6.

## Figur 1

```
1/1                                    31/11
ATG GGT CAC CCG CCG CTG CTG CCG CTG CTG CTG CTG CTC CAC ACC TGC GTC CCA GCC TCT
 M   G   H   P   P   L   L   P   L   L   L   L   L   H   T   C   V   P   A   S
61/21                                  91/31
TGG GGC CTG CGG TGC ATG CAG TGT AAG ACC AAC GGG GAT TGC CGT GTG GAA GAG TGC GCC
 W   G   L   R   C   M   Q   C   K   T   N   G   D   C   R   V   E   E   C   A
121/41                                 151/51
CTG GGA CAG GAC CTC TGC AGG ACC ACG ATC GTG CGC TTG TGG GAA GAA GGA GAA GAG CTG
 L   G   Q   D   L   C   R   T   T   I   V   R   L   W   E   E   G   E   E   L
181/61                                 211/71
GAG CTG GTG GAG AAA AGC TGT ACC CAC TCA GAG AAG ACC AAC AGG ACC CTG AGC TAT CGG
 E   L   V   E   K   S   C   T   H   S   E   K   T   N   R   T   L   S   Y   R
241/81                                 271/91
ACT GGC TTG AAG ATC ACC AGC CTT ACC GAG GTT GTG TGT GGG TTA GAC TTG TGC AAC CAG
 T   G   L   K   I   T   S   L   T   E   V   V   C   G   L   D   L   C   N   Q
301/101                                331/111
GGC AAC TCT GGG CGT CCA AAG GAT GAC CGC CAC CTC CGT GGC TGT GGC TAC CTT CCC GGC
 G   N   S   G   R   P   K   D   D   R   H   L   R   G   C   G   Y   L   P   G
361/121                                391/131
TGC CCG GGC TCC AAT GGT TTC CAC AAC AAC GAC ACC TTC CAC TTC CTG AAA TGC TGC AAC
 C   P   G   S   N   G   F   H   N   N   D   T   F   H   F   L   K   C   C   N
421/141                                451/151
ACC ACC AAA TGC AAC GAG GGC CCA ATC CTG GAG CTT GAA AAT CTG CCG CAG AAT GGC CGC
 T   T   K   C   N   E   G   P   I   L   E   L   E   N   L   P   Q   N   G   R
481/161                                511/171
CAG TGT TAC AGC TGC AAG GGG AAC AGC ACC CAT GGA TGC TCC TCT GAA GAG ACT TTC CTC
 Q   C   Y   S   C   K   G   N   S   T   H   G   C   S   S   E   E   T   F   L
541/181                                571/191
ATT GAC TGC CGA GGC CCC ATG AAT CAA TGT CTG GTA GCC ACC GGC ACT CAC GAA CCG AAA
 I   D   C   R   G   P   M   N   Q   C   L   V   A   T   G   T   H   E   P   K
601/201                                631/211
AAC CAA AGC TAT ATG GTA AGA GGC TGT GCA ACC GCC TCA ATG TGC CAA CAT GCC CAC CTG
 N   Q   S   Y   M   V   R   G   C   A   T   A   S   M   C   Q   H   A   H   L
661/221                                691/231
GGT GAC GCC TTC AGC ATG AAC CAC ATT GAT GTC TCC TGC TGT ACT AAA AGT GGC TGT AAC
 G   D   A   F   S   M   N   H   I   D   V   S   C   C   T   K   S   G   C   N
721/241                                751/251
CAC CCA GAC CTG GAT GTC CAG TAC CGC AGT GGG GCT GCT CCT CAG CCT GGC CCT GCC CAT
 H   P   D   L   D   V   Q   Y   R   S   G   A   A   P   Q   P   G   P   A   H
781/261                                811/271
CTC AGC CTC ACC ATC ACC CTG CTA ATG ACT GCC AGA CTG TGG GGA GGC ACT CTC CTC TGG
 L   S   L   T   I   T   L   L   M   T   A   R   L   W   G   G   T   L   L   W
841/281
ACC TAA
 T   *
```

34

## Figur 2

```
1/1                                      31/11
ATG GGT CAC CCG CCG CTG CTG CCG CTG CTG CTG CTG CTC CAC ACC TGC GTC CCA GCC TCT
M   G   H   P   P   L   L   P   L   L   L   L   L   H   T   C   V   P   A   S
61/21                                    91/31
TGG GGC CTG CGG TGC ATG CAG TGT AAG ACC AAC GGG GAT TGC CGT GTG GAA GAG TGC GCC
W   G   L   R   C   M   Q   C   K   T   N   G   D   C   R   V   E   E   C   A
121/41                                   151/51
CTG GGA CAG GAC CTC TGC AGG ACC ACG ATC GTG CGC TTG TGG GAA GAA GGA GAA GAG CTG
L   G   Q   D   L   C   R   T   T   I   V   R   L   W   E   E   G   E   E   L
181/61                                   211/71
GAG CTG GTG GAG AAA AGC TGT ACC CAC TCA GAG AAG ACC AAC AGG ACC CTG AGC TAT CGG
E   L   V   E   K   S   C   T   H   S   E   K   T   N   R   T   L   S   Y   R
241/81                                   271/91
ACT GGC TTG AAG ATC ACC AGC CTT ACC GAG GTT GTG TGT GGG TTA GAC TTG TGC AAC CAG
T   G   L   K   I   T   S   L   T   E   V   V   C   G   L   D   L   C   N   Q
301/101                                  331/111
GGC AAC TCT GTC CTG GAG CTT GAA AAT CTG CCG CAG AAT GGC CGC CAG TGT TAC AGC TGC
G   N   S   V   L   E   L   E   N   L   P   Q   N   G   R   Q   C   Y   S   C
361/121                                  391/131
AAG GGG AAC AGC ACC CAT GGA TGC TCC TCT GAA GAG ACT TTC CTC ATT GAC TGC CGA GGC
K   G   N   S   T   H   G   C   S   S   E   E   T   F   L   I   D   C   R   G
421/141                                  451/151
CCC ATG AAT CAA TGT CTG GTA GCC ACC GGC ACT CAC GAA CCG AAA AAC CAA AGC TAT ATG
P   M   N   Q   C   L   V   A   T   G   T   H   E   P   K   N   Q   S   Y   M
481/161                                  511/171
GTA AGA GGC TGT GCA ACC GCC TCA ATG TGC CAA CAT GCC CAC CTG GGT GAC GCC TTC AGC
V   R   G   C   A   T   A   S   M   C   Q   H   A   H   L   G   D   A   F   S
541/181                                  571/191
ATG AAC CAC ATT GAT GTC TCC TGC TGT ACT AAA AGT GGC TGT AAC CAC CCA GAC CTG GAT
M   N   H   I   D   V   S   C   C   T   K   S   G   C   N   H   P   D   L   D
601/201                                  631/211
GTC CAG TAC CGC AGT GGG GCT GCT CCT CAG CCT GGC CCT GCC CAT CTC AGC CTC ACC ATC
V   Q   Y   R   S   G   A   A   P   Q   P   G   P   A   H   L   S   L   T   I
661/221                                  691/231
ACC CTG CTA ATG ACT GCC AGA CTG TGG GGA GGC ACT CTC CTC TGG ACC TAA
T   L   L   M   T   A   R   L   W   G   G   T   L   L   W   T   *
```

# Figur 3

```
1/1                                    31/11
ATG GGT CAC CCG CCG CTG CTG CCG CTG CTG CTG CTG CTC CAC ACC TGC GTC CCA GCC TCT
 M   G   H   P   P   L   L   P   L   L   L   L   L   H   T   C   V   P   A   S
61/21                                  91/31
TGG GGC CTG CGG TGC ATG CAG TGT AAG ACC AAC GGG GAT TGC CGT GTG GAA GAG TGC GCC
 W   G   L   R   C   M   Q   C   K   T   N   G   D   C   R   V   E   E   C   A
121/41                                 151/51
CTG GGA CAG GAC CTC TGC AGG ACC ACG ATC GTG CGC TTG TGG GAA GAA GGA GAA GAG CTG
 L   G   Q   D   L   C   R   T   T   I   V   R   L   W   E   E   G   E   E   L
181/61                                 211/71
GAG CTG GTG GAG AAA AGC TGT ACC CAC TCA GAG AAG ACC AAC AGG ACC CTG AGC TAT CGG
 E   L   V   E   K   S   C   T   H   S   E   K   T   N   R   T   L   S   Y   R
241/81                                 271/91
ACT GGC TTG AAG ATC ACC AGC CTT ACC GAG GTT GTG TGT GGG Tta gac tTG TGC AAC CAG
 T   G   L   K   I   T   S   L   T   E   V   V   C   G   L   D   L   C   N   Q
301/101                                331/111
GGC AAC TCT GGC CGG GCT GTC ACC TAT TCC CGA AGC CGT TAC CTC GAA TGC ATT TCC TGT
 G   N   S   G   R   A   V   T   Y   S   R   S   R   Y   L   E   C   I   S   C
361/121                                391/131
GGC TCA TCA GAC ATG AGC TGT GAG AGG GGC CGG CAC CAG AGC CTG CAG TGC CGC AGC CCT
 G   S   S   D   M   S   C   E   R   G   R   H   Q   S   L   Q   C   R   S   P
421/141                                451/151
GAA GAA CAG TGC CTG GAT GTG GTG ACC CAC TGG ATC CAG GAA GGT GAA GAA GTC CTG GAG
 E   E   Q   C   L   D   V   V   T   H   W   I   Q   E   G   E   E   V   L   E
481/161                                511/171
CTT GAA AAT CTG CCG CAG AAT GGC CGC CAG TGT TAC AGC TGC AAG GGG AAC AGC ACC CAT
 L   E   N   L   P   Q   N   G   R   Q   C   Y   S   C   K   G   N   S   T   H
541/181                                571/191
GGA TGC TCC TCT GAA GAG ACT TTC CTC ATT GAC TGC CGA GGC CCC ATG AAT CAA TGT CTG
 G   C   S   S   E   E   T   F   L   I   D   C   R   G   P   M   N   Q   C   L
601/201                                631/211
GTA GCC ACC GGC ACT CAC GAA CCG AAA AAC CAA AGC TAT ATG GTA AGA GGC TGT GCA ACC
 V   A   T   G   T   H   E   P   K   N   Q   S   Y   M   V   R   G   C   A   T
661/221                                691/231
GCC TCA ATG TGC CAA CAT GCC CAC CTG GGT GAC GCC TTC AGC ATG AAC CAC ATT GAT GTC
 A   S   M   C   Q   H   A   H   L   G   D   A   F   S   M   N   H   I   D   V
721/241                                751/251
TCC TGC TGT ACT AAA AGT GGC TGT AAC CAC CCA GAC CTG GAT GTC CAG TAC CGC AGT GGG
 S   C   C   T   K   S   G   C   N   H   P   D   L   D   V   Q   Y   R   S   G
781/261                                811/271
GCT GCT CCT CAG CCT GGC CCT GCC CAT CTC AGC CTC ACC ATC ACC CTG cta atg act gcc
 A   A   P   Q   P   G   P   A   H   L   S   L   T   I   T   L   L   M   T   A
841/281                                871/291
aga ctg tgg gga ggc act ctc ctc tgg acc TAA
 R   L   W   G   G   T   L   L   W   T   *
```

**Figur 4A**

```
              •      20        •      40        •      60           •      80
    1 ATGGGTCACCCGCCGCTGCTGCCGCTGCTGCTGCTGCTCCACACCTGCGTCCCAGCCTCTTGGGGCCTGCGGTGCATGCA 80
      ||||||||||||||||||||||||||||||||||||||||||||||||||||||||||||||||||||||||||||||||
    1 ATGGGTCACCCGCCGCTGCTGCCGCTGCTGCTGCTGCTCCACACCTGCGTCCCAGCCTCTTGGGGCCTGCGGTGCATGCA 80
              •      20        •      40        •      60           •      80
              •     100        •     120        •     140           •     160
   81 GTGTAAGACCAACGGGGATTGCCGTGTGGAAGAGTGCGCCCTGGGACAGGACCTCTGCAGGACCACGATCGTGCGCTTGT 160
      ||||||||||||||||||||||||||||||||||||||||||||||||||||||||||||||||||||||||||||||||
   81 GTGTAAGACCAACGGGGATTGCCGTGTGGAAGAGTGCGCCCTGGGACAGGACCTCTGCAGGACCACGATCGTGCGCTTGT 160
              •     100        •     120        •     140           •     160
              •     180        •     200        •     220           •     240
  161 GGGAAGAAGGAGAAGAGCTGGAGCTGGTGGAGAAAAGCTGTACCCACTCAGAGAAGACCAACAGGACCCTGAGCTATCGG 240
      ||||||||||||||||||||||||||||||||||||||||||||||||||||||||||||||||||||||||||||||||
  161 GGGAAGAAGGAGAAGAGCTGGAGCTGGTGGAGAAAAGCTGTACCCACTCAGAGAAGACCAACAGGACCCTGAGCTATCGG 240
              •     180        •     200        •     220           •     240
              •     260        •     280        •     300
  241 ACTGGCTTGAAGATCACCAGCCTTACCGAGGTTGTGTGTGGGTTAGACTTGTGCAACCAGGGCAACTCT----------- 309
      ||||||||||||||||||||||||||||||||||||||||||||||||||||||||||||||||||||||
  241 ACTGGCTTGAAGATCACCAGCCTTACCGAGGTTGTGTGTGGGTTAGACTTGTGCAACCAGGGCAACTCTGGCCGGGCTGT 320
              •     260        •     280        •     300           •     320


      ----------------------------------------------------------------------------------


  321 CACCTATTCCCGAAGCCGTTACCTCGAATGCATTTCCTGTGGCTCATCAGACATGAGCTGTGAGAGGGGCCGGCACCAGA 400
              •     340        •     360        •     380           •     400

  310 ------------------------------------------------------------------------GGGCGTCCA 318
                                                                             ||||||||||
  401 GCCTGCAGTGCCGCAGCCCTGAAGAACAGTGCCTGGATGTGGTGACCCACTGGATCCAGGAAGGTGAAGAAGGGCGTCCA 480
              •     420        •     440        •     460           •     480
          320          •     340        •     360        •     380           •
  319 AAGGATGACCGCCACCTCCGTGGCTGTGGCTACCTTCCCGGCTGCCCGGGCTCCAATGGTTTCCACAACAACGACACCTT 398
      ||||||||||||||||||||||||||||||||||||||||||||||||||||||||||||||||||||||||||||||||
  481 AAGGATGACCGCCACCTCCGTGGCTGTGGCTACCTTCCCGGCTGCCCGGGCTCCAATGGTTTCCACAACAACGACACCTT 560
              •     500        •     520        •     540           •     560
          400          •     420        •     440        •     460           •
  399 CCACTTCCTGAAATGCTGCAACACCACCAAATGCAACGAGGGCCCAATCCTGGAGCTTGAAAATCTGCCGCAGAATGGCC 478
      ||||||||||||||||||||||||||||||||||||||||||||||||||||||||||||||||||||||||||||||||
  561 CCACTTCCTGAAATGCTGCAACACCACCAAATGCAACGAGGGCCCAATCCTGGAGCTTGAAAATCTGCCGCAGAATGGCC 640
              •     580        •     600        •     620           •     640
          480          •     500        •     520        •     540           •
  479 GCCAGTGTTACAGCTGCAAGGGGAACAGCACCCATGGATGCTCCTCTGAAGAGACTTTCCTCATTGACTGCCGAGGCCCC 558
      ||||||||||||||||||||||||||||||||||||||||||||||||||||||||||||||||||||||||||||||||
  641 GCCAGTGTTACAGCTGCAAGGGGAACAGCACCCATGGATGCTCCTCTGAAGAGACTTTCCTCATTGACTGCCGAGGCCCC 720
              •     660        •     680        •     700           •     720
          560          •     580        •     600        •     620           •
  559 ATGAATCAATGTCTGGTAGCCACCGGCACTCACGAACCGAAAAACCAAAGCTATATGGTAAGAGGCTGTGCAACCGCCTC 638
      ||||||||||||||||||||||||||||||||||||||||||||||||||||||||||||||||||||||||||||||||
  721 ATGAATCAATGTCTGGTAGCCACCGGCACTCACGAACCGAAAAACCAAAGCTATATGGTAAGAGGCTGTGCAACCGCCTC 800
              •     740        •     760        •     780           •     800
          640          •     660        •     680        •     700           •
  639 AATGTGCCAACATGCCCACCTGGGTGACGCCTTCAGCATGAACCACATTGATGTCTCCTGCTGTACTAAAAGTGGCTGTA 718
      ||||||||||||||||||||||||||||||||||||||||||||||||||||||||||||||||||||||||||||||||
  801 AATGTGCCAACATGCCCACCTGGGTGACGCCTTCAGCATGAACCACATTGATGTCTCCTGCTGTACTAAAAGTGGCTGTA 880
              •     820        •     840        •     860           •     880
          720          •     740        •     760        •     780           •
  719 ACCACCCAGACCTGGATGTCCAGTACCGCAGTGGGGCTGCTCCTCAGCCTGGCCCTGCCCATCTCAGCCTCACCATCACC 798
      ||||||||||||||||||||||||||||||||||||||||||||||||||||||||||||||||||||||||||||||||
  881 ACCACCCAGACCTGGATGTCCAGTACCGCAGTGGGGCTGCTCCTCAGCCTGGCCCTGCCCATCTCAGCCTCACCATCACC 960
              •     900        •     920        •     940           •     960
          800          •     820        •     840
  799 CTGCTAATGACTGCCAGACTGTGGGGAGGCACTCTCCTCTGGACCTAA                                846
      |||||||||||||||||||||||||||||||||||||||||||||||
  961 CTGCTAATGACTGCCAGACTGTGGGGAGGCACTCTCCTCTGGACCTAA                                1008
              •     980        •    1000
```

% Identity = 83.9 (846/1008)

## Figur 4B

```
           •        20        •        40        •        60        •        80
 1 MGHPPLLPLLLLLHTCVPASWGLRCMQCKTNGDCRVEECALGQDLCRTTIVRLWEEGEELELVEKSCTHSEKTNRTLSYR 80
   MGHPPLLPLLLLLHTCVPASWGLRCMQCKTNGDCRVEECALGQDLCRTTIVRLWEEGEELELVEKSCTHSEKTNRTLSYR
 1 MGHPPLLPLLLLLHTCVPASWGLRCMQCKTNGDCRVEECALGQDLCRTTIVRLWEEGEELELVEKSCTHSEKTNRTLSYR 80
           •        20        •        40        •        60        •        80
           •       100
81 TGLKITSLTEVVCGLDLCNQGNS-------------------------------------------------GRP 106
   TGLKITSLTEVVCGLDLCNQGNS                                                  GRP
81 TGLKITSLTEVVCGLDLCNQGNSGRAVTYSRSRYLECISCGSSDMSCERGRHQSLQCRSPEEQCLDVVTHWIQEGEEGRP 160
           •       100        •        120        •       140        •       160
           •       120        •       140        •       160        •       180
107 KDDRHLRGCGYLPGCPGSNGFHNNDTFHFLKCCNTTKCNEGPILELENLPQNGRQCYSCKGNSTHGCSSEETFLIDCRGP 186
    KDDRHLRGCGYLPGCPGSNGFHNNDTFHFLKCCNTTKCNEGPILELENLPQNGRQCYSCKGNSTHGCSSEETFLIDCRGP
161 KDDRHLRGCGYLPGCPGSNGFHNNDTFHFLKCCNTTKCNEGPILELENLPQNGRQCYSCKGNSTHGCSSEETFLIDCRGP 240
           •       180        •       200        •       220        •       240
           •       200        •       220        •       240        •       260
187 MNQCLVATGTHEPKNQSYMVRGCATASMCQHAHLGDAFSMNHIDVSCCTKSGCNHPDLDVQYRSGAAPQPGPAHLSLTIT 266
    MNQCLVATGTHEPKNQSYMVRGCATASMCQHAHLGDAFSMNHIDVSCCTKSGCNHPDLDVQYRSGAAPQPGPAHLSLTIT
241 MNQCLVATGTHEPKNQSYMVRGCATASMCQHAHLGDAFSMNHIDVSCCTKSGCNHPDLDVQYRSGAAPQPGPAHLSLTIT 320
           •       260        •       280        •       300        •       320
           •       280
267 LLMTARLWGGTLLWT*                                                               282
    LLMTARLWGGTLLWT*
321 LLMTARLWGGTLLWT*                                                               336
           •
```

% Identity = 83.9 (282/336)

## Figur 5A

```
            •        20         •        40         •        60         •        80
    1 ATGGGTCACCCGCCGCTGCTGCCGCTGCTGCTGCTGCTCCACACCTGCGTCCCAGCCTCTTGGGGCCTGCGGTGCATGCA 80
      |||||||||||||||||||||||||||||||||||||||||||||||||||||||||||||||||||||||||||||||||
    1 ATGGGTCACCCGCCGCTGCTGCCGCTGCTGCTGCTGCTCCACACCTGCGTCCCAGCCTCTTGGGGCCTGCGGTGCATGCA 80
            •        20         •        40         •        60         •        80
            •       100         •       120         •       140         •       160
   81 GTGTAAGACCAACGGGGATTGCCGTGTGGAAGAGTGCGCCCTGGGACAGGACCTCTGCAGGACCACGATCGTGCGCTTGT 160
      |||||||||||||||||||||||||||||||||||||||||||||||||||||||||||||||||||||||||||||||||
   81 GTGTAAGACCAACGGGGATTGCCGTGTGGAAGAGTGCGCCCTGGGACAGGACCTCTGCAGGACCACGATCGTGCGCTTGT 160
            •       100         •       120         •       140         •       160
            •       180         •       200         •       220         •       240
  161 GGGAAGAAGGAGAAGAGCTGGAGCTGGTGGAGAAAAGCTGTACCCACTCAGAGAAGACCAACAGGACCCTGAGCTATCGG 240
      |||||||||||||||||||||||||||||||||||||||||||||||||||||||||||||||||||||||||||||||||
  161 GGGAAGAAGGAGAAGAGCTGGAGCTGGTGGAGAAAAGCTGTACCCACTCAGAGAAGACCAACAGGACCCTGAGCTATCGG 240
            •       180         •       200         •       220         •       240
            •       260         •       280         •       300         •
  241 ACTGGCTTGAAGATCACCAGCCTTACCGAGGTTGTGTGTGGGTTAGACTTGTGCAACCAGGGCAACTCTG---------- 310
      |||||||||||||||||||||||||||||||||||||||||||||||||||||||||||||||||||||||||
  241 ACTGGCTTGAAGATCACCAGCCTTACCGAGGTTGTGTGTGGGTTAGACTTGTGCAACCAGGGCAACTCTGGCCGGGCTGT 320
            •       260         •       280         •       300         •       320


  321 CACCTATTCCCGAAGCCGTTACCTCGAATGCATTTCCTGTGGCTCATCAGACATGAGCTGTGAGAGGGGCCGGCACCAGA 400
            •       340         •       360         •       380         •       400


  401 GCCTGCAGTGCCGCAGCCCTGAAGAACAGTGCCTGGATGTGGTGACCCACTGGATCCAGGAAGGTGAAGAAGGGCGTCCA 480
            •       420         •       440         •       460         •       480


  481 AAGGATGACCGCCACCTCCGTGGCTGTGGCTACCTTCCCGGCTGCCCGGGCTCCAATGGTTTCCACAACAACGACACCTT 560
            •       500         •       520         •       540         •       560
                                                          320         •       340
  311 -----------------------------------------------TCCTGGAGCTTGAAAATCTGCCGCAGAATGGCC 343
                                                      ||||||||||||||||||||||||||||||||
  561 CCACTTCCTGAAATGCTGCAACACCACCAAATGCAACGAGGGCCCAATCCTGGAGCTTGAAAATCTGCCGCAGAATGGCC 640
            •       580         •       600         •       620         •       640
            •       360         •       380         •       400         •       420
  344 GCCAGTGTTACAGCTGCAAGGGGAACAGCACCCATGGATGCTCCTCTGAAGAGACTTTCCTCATTGACTGCCGAGGCCCC 423
      ||||||||||||||||||||||||||||||||||||||||||||||||||||||||||||||||||||||||||||||||
  641 GCCAGTGTTACAGCTGCAAGGGGAACAGCACCCATGGATGCTCCTCTGAAGAGACTTTCCTCATTGACTGCCGAGGCCCC 720
            •       660         •       680         •       700         •       720
            •       440         •       460         •       480         •       500
  424 ATGAATCAATGTCTGGTAGCCACCGGCACTCACGAACCGAAAAACCAAAGCTATATGGTAAGAGGCTGTGCAACCGCCTC 503
      ||||||||||||||||||||||||||||||||||||||||||||||||||||||||||||||||||||||||||||||||
  721 ATGAATCAATGTCTGGTAGCCACCGGCACTCACGAACCGAAAAACCAAAGCTATATGGTAAGAGGCTGTGCAACCGCCTC 800
            •       740         •       760         •       780         •       800
            •       520         •       540         •       560         •       580
  504 AATGTGCCAACATGCCCACCTGGGTGACGCCTTCAGCATGAACCACATTGATGTCTCCTGCTGTACTAAAAGTGGCTGTA 583
      ||||||||||||||||||||||||||||||||||||||||||||||||||||||||||||||||||||||||||||||||
  801 AATGTGCCAACATGCCCACCTGGGTGACGCCTTCAGCATGAACCACATTGATGTCTCCTGCTGTACTAAAAGTGGCTGTA 880
            •       820         •       840         •       860         •       880
            •       600         •       620         •       640         •       660
  584 ACCACCCAGACCTGGATGTCCAGTACCGCAGTGGGGCTGCTCCTCAGCCTGGCCCTGCCCATCTCAGCCTCACCATCACC 663
      ||||||||||||||||||||||||||||||||||||||||||||||||||||||||||||||||||||||||||||||||
  881 ACCACCCAGACCTGGATGTCCAGTACCGCAGTGGGGCTGCTCCTCAGCCTGGCCCTGCCCATCTCAGCCTCACCATCACC 960
            •       900         •       920         •       940         •       960
            •       680         •       700         •
  664 CTGCTAATGACTGCCAGACTGTGGGGAGGCACTCTCCTCTGGACCTAA                                711
      |||||||||||||||||||||||||||||||||||||||||||||||
  961 CTGCTAATGACTGCCAGACTGTGGGGAGGCACTCTCCTCTGGACCTAA                                1008
            •       980         •      1000         •
```

% Identity = 70.5 (711/1008)

**Figur 5B**

```
            •         20        •        40        •        60        •        80
  1 MGHPPLLPLLLLLHTCVPASWGLRCMQCKTNGDCRVEECALGQDLCRTTIVRLWEEGEELELVEKSCTHSEKTNRTLSYR 80
    MGHPPLLPLLLLLHTCVPASWGLRCMQCKTNGDCRVEECALGQDLCRTTIVRLWEEGEELELVEKSCTHSEKTNRTLSYR
  1 MGHPPLLPLLLLLHTCVPASWGLRCMQCKTNGDCRVEECALGQDLCRTTIVRLWEEGEELELVEKSCTHSEKTNRTLSYR 80
            •         20        •        40        •        60        •        80
            •         100
 81 TGLKITSLTEVVCGLDLCNQGNS------------------------------------------------------- 103
    TGLKITSLTEVVCGLDLCNQGNS
 81 TGLKITSLTEVVCGLDLCNQGNSGRAVTYSRSRYLECISCGSSDMSCERGRHQSLQCRSPEEQCLDVVTHWIQEGEEGRP 160
            •         100       •        120       •        140       •        160
                                                •   .     120       •        140
104 ---------------------------------------------------VLELENLPQNGRQCYSCKGNSTHGCSSEETFLIDCRGP 141
                                                  +LELENLPQNGRQCYSCKGNSTHGCSSEETFLIDCRGP
161 KDDRHLRGCGYLPGCPGSNGFHNNDTFHFLKCCNTTKCNEGPILELENLPQNGRQCYSCKGNSTHGCSSEETFLIDCRGP 240
            •         180       •        200       •        220       •        240
            •         160       •        180       •        200       •        220
142 MNQCLVATGTHEPKNQSYMVRGCATASMCQHAHLGDAFSMNHIDVSCCTKSGCNHPDLDVQYRSGAAPQPGPAHLSLTIT 221
    MNQCLVATGTHEPKNQSYMVRGCATASMCQHAHLGDAFSMNHIDVSCCTKSGCNHPDLDVQYRSGAAPQPGPAHLSLTIT
241 MNQCLVATGTHEPKNQSYMVRGCATASMCQHAHLGDAFSMNHIDVSCCTKSGCNHPDLDVQYRSGAAPQPGPAHLSLTIT 320
            •         260       •        280       •        300       •        320
            •
222 LLMTARLWGGTLLWT*                                                              237
    LLMTARLWGGTLLWT*
321 LLMTARLWGGTLLWT*                                                              336
            •

% Identity =  70.2 (236/336)
```

## Figur 6A

```
            •      20        •      40        •      60        •      80
  1 ATGGGTCACCCGCCGCTGCTGCCGCTGCTGCTGCTGCTCCACACCTGCGTCCCAGCCTCTTGGGGCCTGCGGTGCATGCA 80
    ||||||||||||||||||||||||||||||||||||||||||||||||||||||||||||||||||||||||||||||||
  1 ATGGGTCACCCGCCGCTGCTGCCGCTGCTGCTGCTGCTCCACACCTGCGTCCCAGCCTCTTGGGGCCTGCGGTGCATGCA 80
            •      20        •      40        •      60        •      80
            •     100        •     120        •     140        •     160
 81 GTGTAAGACCAACGGGGATTGCCGTGTGGAAGAGTGCGCCCTGGGACAGGACCTCTGCAGGACCACGATCGTGCGCTTGT 160
    ||||||||||||||||||||||||||||||||||||||||||||||||||||||||||||||||||||||||||||||||
 81 GTGTAAGACCAACGGGGATTGCCGTGTGGAAGAGTGCGCCCTGGGACAGGACCTCTGCAGGACCACGATCGTGCGCTTGT 160
            •     100        •     120        •     140        •     160
            •     180        •     200        •     220        •     240
161 GGGAAGAAGGAGAAGAGCTGGAGCTGGTGGAGAAAAGCTGTACCCACTCAGAGAAGACCAACAGGACCCTGAGCTATCGG 240
    ||||||||||||||||||||||||||||||||||||||||||||||||||||||||||||||||||||||||||||||||
161 GGGAAGAAGGAGAAGAGCTGGAGCTGGTGGAGAAAAGCTGTACCCACTCAGAGAAGACCAACAGGACCCTGAGCTATCGG 240
            •     180        •     200        •     220        •     240
            •     260        •     280        •     300        •     320
241 ACTGGCTTGAAGATCACCAGCCTTACCGAGGTTGTGTGTGGGTtagactTGTGCAACCAGGGCAACTCTGGCCGGGCTGT 320
    |||||||||||||||||||||||||||||||||||||||||||      ||||||||||||||||||||||||||||||
241 ACTGGCTTGAAGATCACCAGCCTTACCGAGGTTGTGTGTGGGTTAGACTTGTGCAACCAGGGCAACTCTGGCCGGGCTGT 320
            •     260        •     280        •     300        •     320
            •     340        •     360        •     380        •     400
321 CACCTATTCCCGAAGCCGTTACCTCGAATGCATTTCCTGTGGCTCATCAGACATGAGCTGTGAGAGGGGCCGGCACCAGA 400
    ||||||||||||||||||||||||||||||||||||||||||||||||||||||||||||||||||||||||||||||||
321 CACCTATTCCCGAAGCCGTTACCTCGAATGCATTTCCTGTGGCTCATCAGACATGAGCTGTGAGAGGGGCCGGCACCAGA 400
            •     340        •     360        •     380        •     400
            •     420        •     440        •     460
401 GCCTGCAGTGCCGCAGCCCTGAAGAACAGTGCCTGGATGTGGTGACCCACTGGATCCAGGAAGGTGAAGAAG-------- 472
    |||||||||||||||||||||||||||||||||||||||||||||||||||||||||||||||||||||||||
401 GCCTGCAGTGCCGCAGCCCTGAAGAACAGTGCCTGGATGTGGTGACCCACTGGATCCAGGAAGGTGAAGAAGGGCGTCCA 480
            •     420        •     440        •     460        •     480


    ------------------------------------------------------------------------------

481 AAGGATGACCGCCACCTCCGTGGCTGTGGCTACCTTCCCGGCTGCCCGGGCTCCAATGGTTTCCACAACAACGACACCTT 560
            •     500        •     520        •     540        •     •     560
                                                           480         •     500
473 --------------------------------------------------TCCTGGAGCTTGAAAATCTGCCGCAGAATGGCC 505
                                                      ||||||||||||||||||||||||||||||||||
561 CCACTTCCTGAAATGCTGCAACACCACCAAATGCAACGAGGGCCCAATCCTGGAGCTTGAAAATCTGCCGCAGAATGGCC 640
            •   •  580        •    •  600        •   •  620        •     640
            •     520        •     540        •     560        •     580
506 GCCAGTGTTACAGCTGCAAGGGGAACAGCACCCATGGATGCTCCTCTGAAGAGACTTTCCTCATTGACTGCCGAGGCCCC 585
    ||||||||||||||||||||||||||||||||||||||||||||||||||||||||||||||||||||||||||||||||
641 GCCAGTGTTACAGCTGCAAGGGGAACAGCACCCATGGATGCTCCTCTGAAGAGACTTTCCTCATTGACTGCCGAGGCCCC 720
            •     660        •   •  680        •   •  700        •     720
            •     600        •     620        •     640        •     660
586 ATGAATCAATGTCTGGTAGCCACCGGCACTCACGAACCGAAAAACCAAAGCTATATGGTAAGAGGCTGTGCAACCGCCTC 665
    ||||||||||||||||||||||||||||||||||||||||||||||||||||||||||||||||||||||||||||||||
721 ATGAATCAATGTCTGGTAGCCACCGGCACTCACGAACCGAAAAACCAAAGCTATATGGTAAGAGGCTGTGCAACCGCCTC 800
            •     740        •   •  760        •   •  780        •     800
            •     680        •     700        •     720        •     740
666 AATGTGCCAACATGCCCACCTGGGTGACGCCTTCAGCATGAACCACATTGATGTCTCCTGCTGTACTAAAAGTGGCTGTA 745
    ||||||||||||||||||||||||||||||||||||||||||||||||||||||||||||||||||||||||||||||||
801 AATGTGCCAACATGCCCACCTGGGTGACGCCTTCAGCATGAACCACATTGATGTCTCCTGCTGTACTAAAAGTGGCTGTA 880
            •     820        •   •  840        •   •  860        •     880
            •     760        •     780        •     800        •     820
746 ACCACCCAGACCTGGATGTCCAGTACCGCAGTGGGGCTGCTCCTCAGCCTGGCCCTGCCCATCTCAGCCTCACCATCACC 825
    ||||||||||||||||||||||||||||||||||||||||||||||||||||||||||||||||||||||||||||||||
881 ACCACCCAGACCTGGATGTCCAGTACCGCAGTGGGGCTGCTCCTCAGCCTGGCCCTGCCCATCTCAGCCTCACCATCACC 960
            •     900        •   •  920        •   •  940        •     960
            •     840        •     860        •
826 CTGctaatgactgccagactgtggggaggcactctcctctggaccTAA                                 873
    |||||||||||||||||||||||||||||||||||||||||||||||
961 CTGCTAATGACTGCCAGACTGTGGGGAGGCACTCTCCTCTGGACCTAA                                 1008
            •     980        •    1000
```

% Identity = 86.6 (873/1008)

**Figur 6B**

```
            •         20          •         40          •         60          •         80
  1 MGHPPLLPLLLLLHTCVPASWGLRCMQCKTNGDCRVEECALGQDLCRTTIVRLWEEGEELELVEKSCTHSEKTNRTLSYR 80
    MGHPPLLPLLLLLHTCVPASWGLRCMQCKTNGDCRVEECALGQDLCRTTIVRLWEEGEELELVEKSCTHSEKTNRTLSYR
  1 MGHPPLLPLLLLLHTCVPASWGLRCMQCKTNGDCRVEECALGQDLCRTTIVRLWEEGEELELVEKSCTHSEKTNRTLSYR 80
            •         20          •         40          •         60          •         80
            •        100          •        120          •        140          •
 81 TGLKITSLTEVVCGLDLCNQGNSGRAVTYSRSRYLECISCGSSDMSCERGRHQSLQCRSPEEQCLDVVTHWIQEGEE--- 157
    TGLKITSLTEVVCGLDLCNQGNSGRAVTYSRSRYLECISCGSSDMSCERGRHQSLQCRSPEEQCLDVVTHWIQEGEE
 81 TGLKITSLTEVVCGLDLCNQGNSGRAVTYSRSRYLECISCGSSDMSCERGRHQSLQCRSPEEQCLDVVTHWIQEGEEGRP 160
          •        100          •        120          •        140          •        160
                                                          160          •        180          •
158 ----------------------------------------VLELENLPQNGRQCYSCKGNSTHGCSSEETFLIDCRGP 195
                                            +LELENLPQNGRQCYSCKGNSTHGCSSEETFLIDCRGP
161 KDDRHLRGCGYLPGCPGSNGFHNNDTFHFLKCCNTTKCNEGPILELENLPQNGRQCYSCKGNSTHGCSSEETFLIDCRGP 240
          •        180          •        200          •        220          •        240
        200          •        220          •        240          •        260          •
196 MNQCLVATGTHEPKNQSYMVRGCATASMCQHAHLGDAFSMNHIDVSCCTKSGCNHPDLDVQYRSGAAPQPGPAHLSLTIT 275
    MNQCLVATGTHEPKNQSYMVRGCATASMCQHAHLGDAFSMNHIDVSCCTKSGCNHPDLDVQYRSGAAPQPGPAHLSLTIT
241 MNQCLVATGTHEPKNQSYMVRGCATASMCQHAHLGDAFSMNHIDVSCCTKSGCNHPDLDVQYRSGAAPQPGPAHLSLTIT 320
          •        260          •        280          •        300          •        320
        280          •
276 LLMTARLWGGTLLWT*                                                                  291
    LLMTARLWGGTLLWT*
321 LLMTARLWGGTLLWT*                                                                  336
          •
```

% Identity =   86.3 (290/336)

Figur 7

**Figur 8**

uPAR-antigen

EP 1 386 152 B1

**Figur 9**

**Figur 10**

| | | | | | | | | |
|---|---|---|---|---|---|---|---|---|
| uPARdel4+5 | Q | G | N | S | V | L | E | L |
| uPARdel4 | Q | G | N | S | G | R | P | K |
| uPARdel5 | E | G | E | E | V | L | E | L |

**Figur 11**

Figur 12

EP 1 386 152 B1

| | Ec-uPAR1-283 | uPAR-del4 | uPAR-del45 | uPAR-del5 |
|---|---|---|---|---|
| Kan1-del4 | ++ | ++++ | - | ++ |
| Kan2-del45 | ++ | ++ | ++++ | +++ |
| Huhn1-del4 | - | +++ | + | + |
| Huhn1-del5 | - | - | - | +++ |
| Huhn2-del45 | ++ | ++ | ++ | ++ |
| Mee1-del5 | + | + | + | +++ |

## Figur 13

Sandwich-ELISA mit Zell-Lysaten und Tumorextrakten

| Probe | Verdünnung | Kan. (1)-anti del4/5 IgG | Kan. (1)-anti del4# IgG | Kan. (2)-anti del4/5 IgG | Kan. (2)-anti del4 IgG | Huhn. (1)-anti del4/5 IgG | Huhn. (1)-anti del4 IgG | HU/IIIF10** | HU/HD13** |
|---|---|---|---|---|---|---|---|---|---|
| MaCa-ZLL | | | | | | | | | |
| aMCF-7 | 1:20 | 0,3 | v | 0,25 | v | 0,4 | v | 1,0 | 0,4 |
| T47D | 1:20 | 0,2 | v | 0,35 | 0,25 | 0,5 | 0,2 | 0,35 | v |
| MCF-7 | 1:20 | 0,5 | v | 0,6 | 0,4 | 0,95 | 0,3 | 1,2 | 0,2 |
| HaCaT | 1:20 | v | v | nt | nt | nt | | v | v |
| BT549 | 1:20 | 0,4 | v | nt | nt | nt | nt | 0,4 | 0,15 |
| MaCa-Extrakte | | | | | | | | | |
| 986 | 1:10 | 0,4 | v | 0,3 | v | 0,4 | 0,15 | 1,6 | 1,3 |
| 993 | 1:10 | 1,3 | v | 1,0 | 0,3 | 1,3 | 0,45 | 1,25 | 0,75 |
| 997 | 1:10 | 0,4 | v | 0,3 | v | 0,4 | v | 0,5 | 0,25 |
| 998 | 1:10 | 0,2 | v | 0,15 | v | v | v | 0,8 | 0,3 |
| 999 | 1:10 | v | v | v | v | v | v | v | 0,5 |
| 1000 | 1:10 | 0,25 | v | 0,15 | 0,15 | 0,6 | 0,2 | 0,4 | 0,2 |
| 1001 | 1:10 | 0,6 | v | 0,45 | 0,2 | 0,6 | 0,25 | 0,8 | 0,5 |
| 1016 | 1:10 | 0,6 | v | 0,6 | v | v | v | 0,7 | 0,5 |
| OvCa II | 1:40 | v | v | v | 0,2 | 0,6 | 0,25 | 1,2 | v |
| OvCa III | 1:40 | 0,5 | v | 0,6 | v | v | v | 8,4 | 4,2 |
| OvCa IV | 1:40 | 0,2 | v | v | 0,2 | 0,6 | v | 0,6 | v |
| OvCa V | 1:40 | 0,5 | v | 0,5 | 0,2 | 0,65 | 0,25 | 4,5 | 0,8 |
| EE Stand. | hoch | 1000 | 1700 | 1100 | 1600 | 900 | 1400 | | |
| EE Stand. | niedrig | 150 | 400 | 150 | 150 | 130 | 120 | | |
| EE Background | | 110 | 190 | 130 | 100 | 90 | 90 | | |

* relative uPAR-Konz. bezogen auf entsprechende E.coli uPAR-Variante

** ng/ml

# Kan.(1)-anti-del4 reag. nicht mit E.coli del4/5 im WB + EIA!

Figur 14

EP 1 386 152 B1

| E.coli-Varianten | wt WB | wt EIA*150* /180 | del4 WB | del4 EIA*150* /180 | del4/5 WB | del4/5 EIA*150* /180 | del5 WB | del5 EIA*150* /180 |
|---|---|---|---|---|---|---|---|---|
| **Anti-Kaninchen 1:500** | | | | | | | | |
| del4-I/150 | ++ | +++ | ++++ | +++ | - | - | ++ | +++ |
| del4-II/150 | - | - | - | + | - | + | - | - |
| del4/5-I/150 | + | + | ? | + | ? | ++ | ? | - |
| del4/5-II/150 | ++ | + | ++ | ++ | ++++ | +++ | +++ | - |
| del5-I/90 | nt | - | nt | + | nt | + | nt | - |
| del5-II/150 | + | + | ++ | ++ | +++ | + | +/- | - |
| **Anti-Huhn 1:500** | | | | | | | | |
| del4-I | - | + | +++ | + | + | + | + | + |
| del4-II | - | + | - | (++) | - | ++ | - | +/- |
| del4/5-I | - | - | - | - | - | + | - | +/- |
| del4/5-II | ++ | +++ | ++ | +++ | ++ | +++ | ++ | ++ |
| del5-I | - | + | - | ++ | - | +++ | +++ | + |
| del5-II | +++ | ++ | +/- | +++ | +++ | +++ | +/- | +/- |
| **Anti-Meerschweinchen 1:500** | | | | | | | | |
| del4-I | nt | - | nt | + | nt | + | nt | - |
| del4-II | ++ | - | - | - | - | - | - | - |
| del4/5-I | +++ | - | ++ | + | ++ | + | ++ | - |
| del4/5-II | - | - | - | ++ | +/- | ++ | + | - |
| del5-I | + | - | + | + | + | + | +++ | - |
| del5-II | - | - | - | + | - | + | - | - |
| Extinktionseinheiten <0,500 | - | | | | | | | |
| 0,500-1,000 | + | | | | | | | |
| 1,000-1,500 | ++ | | | | | | | |
| >1,500 | +++ | | | | | | | |

EIA: E.coli-Var. // anti-uPAR-Var.-AS // anti-Spezies-HRP

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10117381

SEQUENZPROTOKOLL

<110> Wilex Biotechnology GmbH

<120> Herstellung polyklonaler, monospezifischer Antikörper gegen die uPAR-Varianten del4, del5 und del4+5 sowie deren Verwendung für diagnostische und therapeutische Zwecke

<130> 24059PWO_DR

<140>
<141>

<150> DE 101 17 381.4
<151> 2001-04-06

<160> 8

<170> PatentIn Ver. 2.1

<210> 1
<211> 846
<212> DNA
<213> Human

<220>
<221> CDS
<222> (1)..(843)

<400> 1

```
atg ggt cac ccg ccg ctg ctg ccg ctg ctg ctg ctg ctc cac acc tgc    48
Met Gly His Pro Pro Leu Leu Pro Leu Leu Leu Leu Leu His Thr Cys
 1               5                  10                  15


gtc cca gcc tct tgg ggc ctg cgg tgc atg cag tgt aag acc aac ggg    96
Val Pro Ala Ser Trp Gly Leu Arg Cys Met Gln Cys Lys Thr Asn Gly
                20                  25                  30


gat tgc cgt gtg gaa gag tgc gcc ctg gga cag gac ctc tgc agg acc   144
Asp Cys Arg Val Glu Glu Cys Ala Leu Gly Gln Asp Leu Cys Arg Thr
            35                  40                  45


acg atc gtg cgc ttg tgg gaa gaa gga gaa gag ctg gag ctg gtg gag   192
Thr Ile Val Arg Leu Trp Glu Glu Gly Glu Glu Leu Glu Leu Val Glu
        50                  55                  60


aaa agc tgt acc cac tca gag aag acc aac agg acc ctg agc tat cgg   240
```

```
Lys Ser Cys Thr His Ser Glu Lys Thr Asn Arg Thr Leu Ser Tyr Arg
65                  70                  75                  80


act ggc ttg aag atc acc agc ctt acc gag gtt gtg tgt ggg tta gac   288
Thr Gly Leu Lys Ile Thr Ser Leu Thr Glu Val Val Cys Gly Leu Asp
                    85                  90                  95


ttg tgc aac cag ggc aac tct ggg cgt cca aag gat gac cgc cac ctc   336
Leu Cys Asn Gln Gly Asn Ser Gly Arg Pro Lys Asp Asp Arg His Leu
                100                 105                 110


cgt ggc tgt ggc tac ctt ccc ggc tgc ccg ggc tcc aat ggt ttc cac   384
Arg Gly Cys Gly Tyr Leu Pro Gly Cys Pro Gly Ser Asn Gly Phe His
            115                 120                 125


aac aac gac acc ttc cac ttc ctg aaa tgc tgc aac acc acc aaa tgc   432
Asn Asn Asp Thr Phe His Phe Leu Lys Cys Cys Asn Thr Thr Lys Cys
        130                 135                 140


aac gag ggc cca atc ctg gag ctt gaa aat ctg ccg cag aat ggc cgc   480
Asn Glu Gly Pro Ile Leu Glu Leu Glu Asn Leu Pro Gln Asn Gly Arg
145                 150                 155                 160


cag tgt tac agc tgc aag ggg aac agc acc cat gga tgc tcc tct gaa   528
Gln Cys Tyr Ser Cys Lys Gly Asn Ser Thr His Gly Cys Ser Ser Glu
                165                 170                 175


gag act ttc ctc att gac tgc cga ggc ccc atg aat caa tgt ctg gta   576
Glu Thr Phe Leu Ile Asp Cys Arg Gly Pro Met Asn Gln Cys Leu Val
                180                 185                 190


gcc acc ggc act cac gaa ccg aaa aac caa agc tat atg gta aga ggc   624
Ala Thr Gly Thr His Glu Pro Lys Asn Gln Ser Tyr Met Val Arg Gly
            195                 200                 205


tgt gca acc gcc tca atg tgc caa cat gcc cac ctg ggt gac gcc ttc   672
Cys Ala Thr Ala Ser Met Cys Gln His Ala His Leu Gly Asp Ala Phe
        210                 215                 220


agc atg aac cac att gat gtc tcc tgc tgt act aaa agt ggc tgt aac   720
Ser Met Asn His Ile Asp Val Ser Cys Cys Thr Lys Ser Gly Cys Asn
225                 230                 235                 240


cac cca gac ctg gat gtc cag tac cgc agt ggg gct gct cct cag cct   768
His Pro Asp Leu Asp Val Gln Tyr Arg Ser Gly Ala Ala Pro Gln Pro
                245                 250                 255


ggc cct gcc cat ctc agc ctc acc atc acc ctg cta atg act gcc aga   816
```

```
Gly Pro Ala His Leu Ser Leu Thr Ile Thr Leu Leu Met Thr Ala Arg
            260                 265                 270

ctg tgg gga ggc act ctc ctc tgg acc taa                          846
Leu Trp Gly Gly Thr Leu Leu Trp Thr
        275                 280
```

<210> 2
<211> 281
<212> PRT
<213> Human

<400> 2

```
Met Gly His Pro Pro Leu Leu Pro Leu Leu Leu Leu Leu His Thr Cys
 1              5               10                 15

Val Pro Ala Ser Trp Gly Leu Arg Cys Met Gln Cys Lys Thr Asn Gly
            20                  25                 30

Asp Cys Arg Val Glu Glu Cys Ala Leu Gly Gln Asp Leu Cys Arg Thr
        35                  40                  45

Thr Ile Val Arg Leu Trp Glu Glu Gly Glu Glu Leu Glu Leu Val Glu
        50                  55                  60

Lys Ser Cys Thr His Ser Glu Lys Thr Asn Arg Thr Leu Ser Tyr Arg
 65             70                  75                      80

Thr Gly Leu Lys Ile Thr Ser Leu Thr Glu Val Val Cys Gly Leu Asp
                85                  90                  95

Leu Cys Asn Gln Gly Asn Ser Gly Arg Pro Lys Asp Asp Arg His Leu
            100                 105                 110

Arg Gly Cys Gly Tyr Leu Pro Gly Cys Pro Gly Ser Asn Gly Phe His
        115                 120                 125

Asn Asn Asp Thr Phe His Phe Leu Lys Cys Cys Asn Thr Thr Lys Cys
    130                 135                 140

Asn Glu Gly Pro Ile Leu Glu Leu Glu Asn Leu Pro Gln Asn Gly Arg
145                 150                 155                 160

Gln Cys Tyr Ser Cys Lys Gly Asn Ser Thr His Gly Cys Ser Ser Glu
            165                 170                 175

Glu Thr Phe Leu Ile Asp Cys Arg Gly Pro Met Asn Gln Cys Leu Val
```

12

```
                180                    185                    190

    Ala Thr Gly Thr His Glu Pro Lys Asn Gln Ser Tyr Met Val Arg Gly
            195                200                205

    Cys Ala Thr Ala Ser Met Cys Gln His Ala His Leu Gly Asp Ala Phe
            210                215                220

    Ser Met Asn His Ile Asp Val Ser Cys Cys Thr Lys Ser Gly Cys Asn
    225                230                235                240

    His Pro Asp Leu Asp Val Gln Tyr Arg Ser Gly Ala Ala Pro Gln Pro
                   245                250                255

    Gly Pro Ala His Leu Ser Leu Thr Ile Thr Leu Leu Met Thr Ala Arg
                260                265                270

    Leu Trp Gly Gly Thr Leu Leu Trp Thr
            275                280
```

<210> 3
<211> 711
<212> DNA
<213> Human

<220>
<221> CDS
<222> (1)..(708)

<400> 3

```
atg ggt cac ccg ccg ctg ctg ccg ctg ctg ctg ctg ctc cac acc tgc    48
Met Gly His Pro Pro Leu Leu Pro Leu Leu Leu Leu Leu His Thr Cys
 1               5                  10                  15

gtc cca gcc tct tgg ggc ctg cgg tgc atg cag tgt aag acc aac ggg    96
Val Pro Ala Ser Trp Gly Leu Arg Cys Met Gln Cys Lys Thr Asn Gly
             20                  25                  30

gat tgc cgt gtg gaa gag tgc gcc ctg gga cag gac ctc tgc agg acc   144
Asp Cys Arg Val Glu Glu Cys Ala Leu Gly Gln Asp Leu Cys Arg Thr
         35                  40                  45

acg atc gtg cgc ttg tgg gaa gaa gga gaa gag ctg gag ctg gtg gag   192
Thr Ile Val Arg Leu Trp Glu Glu Gly Glu Glu Leu Glu Leu Val Glu
     50                  55                  60
```

```
aaa agc tgt acc cac tca gag aag acc aac agg acc ctg agc tat cgg      240
Lys Ser Cys Thr His Ser Glu Lys Thr Asn Arg Thr Leu Ser Tyr Arg
 65                      70                  75                  80

act ggc ttg aag atc acc agc ctt acc gag gtt gtg tgt ggg tta gac      288
Thr Gly Leu Lys Ile Thr Ser Leu Thr Glu Val Val Cys Gly Leu Asp
                        85                  90                  95

ttg tgc aac cag ggc aac tct gtc ctg gag ctt gaa aat ctg ccg cag      336
Leu Cys Asn Gln Gly Asn Ser Val Leu Glu Leu Glu Asn Leu Pro Gln
                100                 105                 110

aat ggc cgc cag tgt tac agc tgc aag ggg aac agc acc cat gga tgc      384
Asn Gly Arg Gln Cys Tyr Ser Cys Lys Gly Asn Ser Thr His Gly Cys
            115                 120                 125

tcc tct gaa gag act ttc ctc att gac tgc cga ggc ccc atg aat caa      432
Ser Ser Glu Glu Thr Phe Leu Ile Asp Cys Arg Gly Pro Met Asn Gln
        130                 135                 140

tgt ctg gta gcc acc ggc act cac gaa ccg aaa aac caa agc tat atg      480
Cys Leu Val Ala Thr Gly Thr His Glu Pro Lys Asn Gln Ser Tyr Met
145                 150                 155                 160

gta aga ggc tgt gca acc gcc tca atg tgc caa cat gcc cac ctg ggt      528
Val Arg Gly Cys Ala Thr Ala Ser Met Cys Gln His Ala His Leu Gly
                165                 170                 175

gac gcc ttc agc atg aac cac att gat gtc tcc tgc tgt act aaa agt      576
Asp Ala Phe Ser Met Asn His Ile Asp Val Ser Cys Cys Thr Lys Ser
                180                 185                 190

ggc tgt aac cac cca gac ctg gat gtc cag tac cgc agt ggg gct gct      624
Gly Cys Asn His Pro Asp Leu Asp Val Gln Tyr Arg Ser Gly Ala Ala
                195                 200                 205

cct cag cct ggc cct gcc cat ctc agc ctc acc atc acc ctg cta atg      672
Pro Gln Pro Gly Pro Ala His Leu Ser Leu Thr Ile Thr Leu Leu Met
        210                 215                 220

act gcc aga ctg tgg gga ggc act ctc ctc tgg acc taa                  711
Thr Ala Arg Leu Trp Gly Gly Thr Leu Leu Trp Thr
225                 230                 235
```

<210> 4

<211> 236
<212> PRT
<213> Human

<400> 4

```
Met Gly His Pro Pro Leu Leu Pro Leu Leu Leu Leu Leu His Thr Cys
 1               5                  10                  15

Val Pro Ala Ser Trp Gly Leu Arg Cys Met Gln Cys Lys Thr Asn Gly
                20                  25                  30

Asp Cys Arg Val Glu Glu Cys Ala Leu Gly Gln Asp Leu Cys Arg Thr
            35                  40                  45

Thr Ile Val Arg Leu Trp Glu Glu Gly Glu Glu Leu Glu Leu Val Glu
        50                  55                  60

Lys Ser Cys Thr His Ser Glu Lys Thr Asn Arg Thr Leu Ser Tyr Arg
65                  70                  75                  80

Thr Gly Leu Lys Ile Thr Ser Leu Thr Glu Val Val Cys Gly Leu Asp
                85                  90                  95

Leu Cys Asn Gln Gly Asn Ser Val Leu Glu Leu Glu Asn Leu Pro Gln
            100                 105                 110

Asn Gly Arg Gln Cys Tyr Ser Cys Lys Gly Asn Ser Thr His Gly Cys
            115                 120                 125

Ser Ser Glu Glu Thr Phe Leu Ile Asp Cys Arg Gly Pro Met Asn Gln
    130                 135                 140

Cys Leu Val Ala Thr Gly Thr His Glu Pro Lys Asn Gln Ser Tyr Met
145                 150                 155                 160

Val Arg Gly Cys Ala Thr Ala Ser Met Cys Gln His Ala His Leu Gly
                165                 170                 175

Asp Ala Phe Ser Met Asn His Ile Asp Val Ser Cys Cys Thr Lys Ser
            180                 185                 190

Gly Cys Asn His Pro Asp Leu Asp Val Gln Tyr Arg Ser Gly Ala Ala
            195                 200                 205

Pro Gln Pro Gly Pro Ala His Leu Ser Leu Thr Ile Thr Leu Leu Met
    210                 215                 220

Thr Ala Arg Leu Trp Gly Gly Thr Leu Leu Trp Thr
225                 230                 235
```

<210> 5

<211> 873
<212> DNA
<213> Human

<220>
<221> CDS
<222> (1) .. (870)

<400> 5

```
atg ggt cac ccg ccg ctg ctg ccg ctg ctg ctg ctg ctc cac acc tgc     48
Met Gly His Pro Pro Leu Leu Pro Leu Leu Leu Leu Leu His Thr Cys
1               5                   10                  15

gtc cca gcc tct tgg ggc ctg cgg tgc atg cag tgt aag acc aac ggg     96
Val Pro Ala Ser Trp Gly Leu Arg Cys Met Gln Cys Lys Thr Asn Gly
                20                  25                  30

gat tgc cgt gtg gaa gag tgc gcc ctg gga cag gac ctc tgc agg acc    144
Asp Cys Arg Val Glu Glu Cys Ala Leu Gly Gln Asp Leu Cys Arg Thr
            35                  40                  45

acg atc gtg cgc ttg tgg gaa gaa gga gaa gag ctg gag ctg gtg gag    192
Thr Ile Val Arg Leu Trp Glu Glu Gly Glu Glu Leu Glu Leu Val Glu
        50                  55                  60

aaa agc tgt acc cac tca gag aag acc aac agg acc ctg agc tat cgg    240
Lys Ser Cys Thr His Ser Glu Lys Thr Asn Arg Thr Leu Ser Tyr Arg
65                  70                  75                  80

act ggc ttg aag atc acc agc ctt acc gag gtt gtg tgt ggg tta gac    288
Thr Gly Leu Lys Ile Thr Ser Leu Thr Glu Val Val Cys Gly Leu Asp
                85                  90                  95

ttg tgc aac cag ggc aac tct ggc cgg gct gtc acc tat tcc cga agc    336
Leu Cys Asn Gln Gly Asn Ser Gly Arg Ala Val Thr Tyr Ser Arg Ser
                100                 105                 110

cgt tac ctc gaa tgc att tcc tgt ggc tca tca gac atg agc tgt gag    384
Arg Tyr Leu Glu Cys Ile Ser Cys Gly Ser Ser Asp Met Ser Cys Glu
                115                 120                 125

agg ggc cgg cac cag agc ctg cag tgc cgc agc cct gaa gaa cag tgc    432
Arg Gly Arg His Gln Ser Leu Gln Cys Arg Ser Pro Glu Glu Gln Cys
        130                 135                 140
```

```
ctg gat gtg gtg acc cac tgg atc cag gaa ggt gaa gaa gtc ctg gag    480
Leu Asp Val Val Thr His Trp Ile Gln Glu Gly Glu Glu Val Leu Glu
145             150             155             160

ctt gaa aat ctg ccg cag aat ggc cgc cag tgt tac agc tgc aag ggg    528
Leu Glu Asn Leu Pro Gln Asn Gly Arg Gln Cys Tyr Ser Cys Lys Gly
            165             170             175

aac agc acc cat gga tgc tcc tct gaa gag act ttc ctc att gac tgc    576
Asn Ser Thr His Gly Cys Ser Ser Glu Glu Thr Phe Leu Ile Asp Cys
            180             185             190

cga ggc ccc atg aat caa tgt ctg gta gcc acc ggc act cac gaa ccg    624
Arg Gly Pro Met Asn Gln Cys Leu Val Ala Thr Gly Thr His Glu Pro
            195             200             205

aaa aac caa agc tat atg gta aga ggc tgt gca acc gcc tca atg tgc    672
Lys Asn Gln Ser Tyr Met Val Arg Gly Cys Ala Thr Ala Ser Met Cys
        210             215             220

caa cat gcc cac ctg ggt gac gcc ttc agc atg aac cac att gat gtc    720
Gln His Ala His Leu Gly Asp Ala Phe Ser Met Asn His Ile Asp Val
225             230             235             240

tcc tgc tgt act aaa agt ggc tgt aac cac cca gac ctg gat gtc cag    768
Ser Cys Cys Thr Lys Ser Gly Cys Asn His Pro Asp Leu Asp Val Gln
            245             250             255

tac cgc agt ggg gct gct cct cag cct ggc cct gcc cat ctc agc ctc    816
Tyr Arg Ser Gly Ala Ala Pro Gln Pro Gly Pro Ala His Leu Ser Leu
            260             265             270

acc atc acc ctg cta atg act gcc aga ctg tgg gga ggc act ctc ctc    864
Thr Ile Thr Leu Leu Met Thr Ala Arg Leu Trp Gly Gly Thr Leu Leu
            275             280             285

tgg acc taa                                                        873
Trp Thr
        290
```

```
<210> 6
<211> 290
<212> PRT
<213> Human

<400> 6
```

Met Gly His Pro Pro Leu Leu Pro Leu Leu Leu Leu Leu His Thr Cys

**EP 1 386 152 B1**

```
              1                   5                    10                   15

        Val Pro Ala Ser Trp Gly Leu Arg Cys Met Gln Cys Lys Thr Asn Gly
                    20                  25                  30

        Asp Cys Arg Val Glu Glu Cys Ala Leu Gly Gln Asp Leu Cys Arg Thr
                    35                  40                  45

        Thr Ile Val Arg Leu Trp Glu Glu Gly Glu Glu Leu Glu Leu Val Glu
                    50                  55                  60

        Lys Ser Cys Thr His Ser Glu Lys Thr Asn Arg Thr Leu Ser Tyr Arg
            65                  70                  75                  80

        Thr Gly Leu Lys Ile Thr Ser Leu Thr Glu Val Val Cys Gly Leu Asp
                        85                  90                  95

        Leu Cys Asn Gln Gly Asn Ser Gly Arg Ala Val Thr Tyr Ser Arg Ser
                    100                 105                 110

        Arg Tyr Leu Glu Cys Ile Ser Cys Gly Ser Ser Asp Met Ser Cys Glu
                    115                 120                 125

        Arg Gly Arg His Gln Ser Leu Gln Cys Arg Ser Pro Glu Glu Gln Cys
            130                 135                 140

        Leu Asp Val Val Thr His Trp Ile Gln Glu Gly Glu Glu Val Leu Glu
        145                 150                 155                 160

        Leu Glu Asn Leu Pro Gln Asn Gly Arg Gln Cys Tyr Ser Cys Lys Gly
                    165                 170                 175

        Asn Ser Thr His Gly Cys Ser Ser Glu Glu Thr Phe Leu Ile Asp Cys
                    180                 185                 190

        Arg Gly Pro Met Asn Gln Cys Leu Val Ala Thr Gly Thr His Glu Pro
                    195                 200                 205

        Lys Asn Gln Ser Tyr Met Val Arg Gly Cys Ala Thr Ala Ser Met Cys
            210                 215                 220

        Gln His Ala His Leu Gly Asp Ala Phe Ser Met Asn His Ile Asp Val
        225                 230                 235                 240

        Ser Cys Cys Thr Lys Ser Gly Cys Asn His Pro Asp Leu Asp Val Gln
                    245                 250                 255

        Tyr Arg Ser Gly Ala Ala Pro Gln Pro Gly Pro Ala His Leu Ser Leu
```

21

                    260                     265                     270

    Thr Ile Thr Leu Leu Met Thr Ala Arg Leu Trp Gly Gly Thr Leu Leu
            275                     280                     285

    Trp Thr
        290


<210> 7
<211> 1008
<212> DNA
<213> Human

<220>
<221> CDS
<222> (1)..(1005)

<400> 7

```
atg ggt cac ccg ccg ctg ctg ccg ctg ctg ctg ctg ctc cac acc tgc    48
Met Gly His Pro Pro Leu Leu Pro Leu Leu Leu Leu Leu His Thr Cys
 1               5                  10                  15

gtc cca gcc tct tgg ggc ctg cgg tgc atg cag tgt aag acc aac ggg    96
Val Pro Ala Ser Trp Gly Leu Arg Cys Met Gln Cys Lys Thr Asn Gly
                20                  25                  30

gat tgc cgt gtg gaa gag tgc gcc ctg gga cag gac ctc tgc agg acc   144
Asp Cys Arg Val Glu Glu Cys Ala Leu Gly Gln Asp Leu Cys Arg Thr
            35                  40                  45

acg atc gtg cgc ttg tgg gaa gaa gga gaa gag ctg gag ctg gtg gag   192
Thr Ile Val Arg Leu Trp Glu Glu Gly Glu Glu Leu Glu Leu Val Glu
        50                  55                  60

aaa agc tgt acc cac tca gag aag acc aac agg acc ctg agc tat cgg   240
Lys Ser Cys Thr His Ser Glu Lys Thr Asn Arg Thr Leu Ser Tyr Arg
65                  70                  75                  80

act ggc ttg aag atc acc agc ctt acc gag gtt gtg tgt ggg tta gac   288
Thr Gly Leu Lys Ile Thr Ser Leu Thr Glu Val Val Cys Gly Leu Asp
                85                  90                  95

ttg tgc aac cag ggc aac tct ggc cgg gct gtc acc tat tcc cga agc   336
Leu Cys Asn Gln Gly Asn Ser Gly Arg Ala Val Thr Tyr Ser Arg Ser
                100                 105                 110
```

```
cgt tac ctc gaa tgc att tcc tgt ggc tca tca gac atg agc tgt gag    384
Arg Tyr Leu Glu Cys Ile Ser Cys Gly Ser Ser Asp Met Ser Cys Glu
        115             120             125


agg ggc cgg cac cag agc ctg cag tgc cgc agc cct gaa gaa cag tgc    432
Arg Gly Arg His Gln Ser Leu Gln Cys Arg Ser Pro Glu Glu Gln Cys
    130             135             140


ctg gat gtg gtg acc cac tgg atc cag gaa ggt gaa gaa ggg cgt cca    480
Leu Asp Val Val Thr His Trp Ile Gln Glu Gly Glu Glu Gly Arg Pro
145             150             155             160


aag gat gac cgc cac ctc cgt ggc tgt ggc tac ctt ccc ggc tgc ccg    528
Lys Asp Asp Arg His Leu Arg Gly Cys Gly Tyr Leu Pro Gly Cys Pro
                165             170             175


ggc tcc aat ggt ttc cac aac aac gac acc ttc cac ttc ctg aaa tgc    576
Gly Ser Asn Gly Phe His Asn Asn Asp Thr Phe His Phe Leu Lys Cys
        180             185             190


tgc aac acc acc aaa tgc aac gag ggc cca atc ctg gag ctt gaa aat    624
Cys Asn Thr Thr Lys Cys Asn Glu Gly Pro Ile Leu Glu Leu Glu Asn
        195             200             205


ctg ccg cag aat ggc cgc cag tgt tac agc tgc aag ggg aac agc acc    672
Leu Pro Gln Asn Gly Arg Gln Cys Tyr Ser Cys Lys Gly Asn Ser Thr
    210             215             220


cat gga tgc tcc tct gaa gag act ttc ctc att gac tgc cga ggc ccc    720
His Gly Cys Ser Ser Glu Glu Thr Phe Leu Ile Asp Cys Arg Gly Pro
225             230             235             240


atg aat caa tgt ctg gta gcc acc ggc act cac gaa ccg aaa aac caa    768
Met Asn Gln Cys Leu Val Ala Thr Gly Thr His Glu Pro Lys Asn Gln
                245             250             255


agc tat atg gta aga ggc tgt gca acc gcc tca atg tgc caa cat gcc    816
Ser Tyr Met Val Arg Gly Cys Ala Thr Ala Ser Met Cys Gln His Ala
                260             265             270


cac ctg ggt gac gcc ttc agc atg aac cac att gat gtc tcc tgc tgt    864
His Leu Gly Asp Ala Phe Ser Met Asn His Ile Asp Val Ser Cys Cys
        275             280             285


act aaa agt ggc tgt aac cac cca gac ctg gat gtc cag tac cgc agt    912
Thr Lys Ser Gly Cys Asn His Pro Asp Leu Asp Val Gln Tyr Arg Ser
    290             295             300
```

24

```
ggg gct gct cct cag cct ggc cct gcc cat ctc agc ctc acc atc acc    960
Gly Ala Ala Pro Gln Pro Gly Pro Ala His Leu Ser Leu Thr Ile Thr
305             310             315             320

ctg cta atg act gcc aga ctg tgg gga ggc act ctc ctc tgg acc taa   1008
Leu Leu Met Thr Ala Arg Leu Trp Gly Gly Thr Leu Leu Trp Thr
                325             330             335
```

<210> 8
<211> 335
<212> PRT
<213> Human

<400> 8

```
Met Gly His Pro Pro Leu Leu Pro Leu Leu Leu Leu Leu His Thr Cys
 1               5               10                  15

Val Pro Ala Ser Trp Gly Leu Arg Cys Met Gln Cys Lys Thr Asn Gly
                20                  25                  30

Asp Cys Arg Val Glu Glu Cys Ala Leu Gly Gln Asp Leu Cys Arg Thr
        35                  40                  45

Thr Ile Val Arg Leu Trp Glu Glu Gly Glu Glu Leu Glu Leu Val Glu
    50                  55                  60

Lys Ser Cys Thr His Ser Glu Lys Thr Asn Arg Thr Leu Ser Tyr Arg
65                  70                  75                  80

Thr Gly Leu Lys Ile Thr Ser Leu Thr Glu Val Val Cys Gly Leu Asp
                85                  90                  95

Leu Cys Asn Gln Gly Asn Ser Gly Arg Ala Val Thr Tyr Ser Arg Ser
        100                 105                 110

Arg Tyr Leu Glu Cys Ile Ser Cys Gly Ser Ser Asp Met Ser Cys Glu
        115                 120                 125

Arg Gly Arg His Gln Ser Leu Gln Cys Arg Ser Pro Glu Glu Gln Cys
    130                 135                 140

Leu Asp Val Val Thr His Trp Ile Gln Glu Gly Glu Glu Gly Arg Pro
145                 150                 155                 160

Lys Asp Asp Arg His Leu Arg Gly Cys Gly Tyr Leu Pro Gly Cys Pro
                165                 170                 175
```

```
Gly Ser Asn Gly Phe His Asn Asn Asp Thr Phe His Phe Leu Lys Cys
            180             185             190

Cys Asn Thr Thr Lys Cys Asn Glu Gly Pro Ile Leu Glu Leu Glu Asn
            195             200             205

Leu Pro Gln Asn Gly Arg Gln Cys Tyr Ser Cys Lys Gly Asn Ser Thr
    210             215             220

His Gly Cys Ser Ser Glu Glu Thr Phe Leu Ile Asp Cys Arg Gly Pro
225             230             235             240

Met Asn Gln Cys Leu Val Ala Thr Gly Thr His Glu Pro Lys Asn Gln
            245             250             255

Ser Tyr Met Val Arg Gly Cys Ala Thr Ala Ser Met Cys Gln His Ala
            260             265             270

His Leu Gly Asp Ala Phe Ser Met Asn His Ile Asp Val Ser Cys Cys
            275             280             285

Thr Lys Ser Gly Cys Asn His Pro Asp Leu Asp Val Gln Tyr Arg Ser
    290             295             300

Gly Ala Ala Pro Gln Pro Gly Pro Ala His Leu Ser Leu Thr Ile Thr
305             310             315             320

Leu Leu Met Thr Ala Arg Leu Trp Gly Gly Thr Leu Leu Trp Thr
            325             330             335
```